# EUROPEAN PATENT SPECIFICATION

(11) **EP 4 346 822 B1**
(45) Date of publication and mention of the grant of the patent: **25.02.2026**
(21) Application number: 22740584.2
(22) Date of filing: 02.06.2022
(51) Int. Cl.: A61K 31/4709, A61K 31/4725, A61K 31/675, A61K 45/06, A61K 9/00, A61K 9/06, A61K 9/08, A61K 9/10, A61K 9/107, A61K 9/20, A61K 9/48, A61K 9/70, A61P 1/16, A61P 43/00

(54) **COMPOUND AND COMPOSITION FOR TREATING NON-ALCOHOLIC STEATOHEPATITIS**
VERBINDUNG UND ZUSAMMENSETZUNG ZUR BEHANDLUNG VON DER NICHT-ALKOHOLISCHEN FETTLEBERENTZÜNDUNG
COMPOSÉ ET COMPOSITION POUR LE TRAITEMENT DE LA STÉATOHÉPATITE NON ALCOOLIQUE

(30) Priority: 04.06.2021 US 202163197038 P
(43) Date of publication of application: 10.04.2024
(73) Proprietor: GILEAD SCIENCES, INC., Foster City, California 94404 (US)
(72) Inventor: BRECKENRIDGE, David G., San Mateo, California 94403 (US); BUDAS, Grant R., Foster City, California 94404 (US); GUPTA, Ruchi, Belmont, California 94002 (US); TAYLOR, James G., Foster City, California 94404 (US); WARR, Matthew R., Westfield, New Jersey 07090 (US); WRIGHT, Nathan E., San Diego, California 92131 (US)
(74) Representative: Müller, Christian Stefan Gerd
(86) International application number: PCT/US2022/031963
(87) International publication number: WO 2022/256529

(56) References cited:
- WO-A1-2017/007689
- WO-A1-2017/007694
- WO-A1-2018/005435
- WO-A1-2020/252151
- BATES JAMIE ET AL: "Acetyl-CoA carboxylase inhibition disrupts metabolic reprogramming during hepatic stellate cell activation", JOURNAL OF HEPATOLOGY, ELSEVIER, AMSTERDAM, NL, vol. 73, no. 4, 4 May 2020 (2020-05-04), pages 896 - 905, XP086258560, ISSN: 0168-8278, [retrieved on 20200504], DOI: 10.1016/J.JHEP.2020.04.037

## Description

This application claims the benefit under 35 U.S.C. § 119(e) of U.S. Provisional Application No. 63/197,038, filed on June 4, 2021.

### Field

The present disclosure relates generally to methods of using modulators of COT (cancer Osaka thyroid) for treating, stabilizing, or lessening the severity or progression liver disease, in particular, nonalcoholic steatohepatitis (NASH).

### Background

NASH (sometimes called steatonecrosis) is diagnosed most often in patients between 40 and 60 years but can occur in all age groups. Many affected patients have obesity, type 2 diabetes mellitus (or glucose intolerance), dyslipidemia, and/or metabolic syndrome. Histologically, NASH is indistinguishable from alcoholic hepatitis. Thus to diagnosis NASH, underlying alcohol use must be ruled out. Differentiating simple steatosis from NASH can be difficult, and elevated liver enzymes are not a sensitive predictor for identifying NASH. The presence of metabolic syndrome (obesity, dyslipidemia, hypertension, and glucose intolerance) increases the likelihood that a patient has NASH rather than simple steatosis. Pathogenesis is poorly understood but seems to be linked to insulin resistance (e.g., as in obesity or metabolic syndrome). Most patients are asymptomatic. Although noninvasive diagnostic tests are usually sufficient, liver biopsy remains the gold standard. Treatment includes elimination of causes and risk factors; new therapies are rapidly emerging but still in the clinical trial phase.

Lifestyle modification and associated weight loss can improve NASH but are not always sufficient and sustained results are difficult to obtain. Hence, there is an urgent need for pharmacological treatment.

### Summary of the present invention

The present invention is defined by the appended claims.

In addition, any references to methods of treatment in the subsequent paragraphs of this description are to be interpreted as references to the compounds, pharmaceutical compositions, and/or medicaments of the present invention for use in methods of treatment of the human (or animal) body by therapy (or diagnosis).

Cancer Osaka thyroid (COT) protein is a serine/threonine kinase that is a member of the MAP kinase kinase kinase (MAP3K) family. It is also known as "TPL2" (tumor progression locus), "MAP3K8" (mitogen-activated protein kinase kinase kinase 8) or "EST" (Ewing sarcoma transformant). COT was identified by its oncogenic transforming activity in cells and has been shown to regulate oncogenic and inflammatory pathways.

As shown herein, TPL2 (or COT) inhibition reduces fibrosis in a choline-deficient high-fat diet (CDHFD) NASH animal model.

The present invention provides a compound having the formula 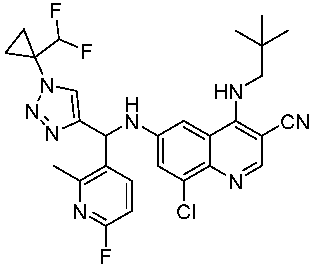 or a pharmaceutically acceptable salt thereof, or a pharmaceutical composition comprising the compound or a pharmaceutically acceptable salt thereof and one or more pharmaceutically acceptable vehicles selected from carriers, adjuvants and excipients, for use in a method for treating non-alcoholic steatohepatitis (NASH) in a subject.

In certain embodiments, the use further comprises administering an effective amount of an ACC inhibitor.

### Brief Description of Drawings

**FIG. 1****,** panels A-C, show PSR of liver tissue from subjects administered vehicle, Compound A (TPL2i), and Compound B (ACCi) in a rat choline-deficient high-fat diet (CDHFD) model.
**FIG. 2** shows PSR as percent area of liver tissue from subjects administered Compound A (TPL2i), Compound B (ACCi), and vehicle in a rat choline-deficient high-fat diet (CDHFD) model. Data presented as mean ± SEM. ****p < 0.0001, ***p < 0.001, **p < 0.01, *p < 0.05 vs. vehicle; #p<0.01, ###p < 0.001 vs. ACCi; ^p<0.05 vs. CVC; one-way ANOVA, Tukey's post hoc test.
**FIG. 3** shows hydroxyproline (OH-P) content from subjects administered Compound A (TPL2i), Compound B (ACCi) and combinations thereof in a rat choline deficient high fat diet (CDHFD) model. Data presented as mean ± SEM. ****p < 0.0001, ***p < 0.001, **p < 0.01, *p < 0.05 vs. vehicle; #p<0.01, ###p < 0.001 vs. ACCi; ^p<0.05 vs. CVC; one-way ANOVA, Tukey's post hoc test.
**FIG. 4** shows a heatmap of liver cytokine levels measured by ELISA in a rat choline deficient high fat diet (CDHFD) model. ****p < 0.0001, ***p < 0.001, **p < 0.01, *p < 0.05 vs. vehicle; one-way ANOVA, Tukey's post hoc test.
**FIG. 5** shows the effect of Compound A, Compound B, and the combination of Compound A and Compound B on body weight in the Gubra amylin NASH (GAN) mouse model. CVC = Cenicriviroc, used as a control (100 mg/Kg QD). Vehicle = 5% TPGS/10% PVP-VA/85% 0.01M HCl. Data presented as mean ± SEM.
**FIG. 6** shows the effect of Compound A, Compound B, and the combination of Compound A and Compound B on whole body fat mass after in the Gubra amylin NASH (GAN) mouse model. CVC = Cenicriviroc, used as a control. DIO-NASH-vehicle = 5% TPGS/10% PVP-VA/85% 0.01M HCl. Data presented as mean ± SEM.
**FIG. 7** shows picrosirius red staining (PSR) from liver tissue after administration of vehicle (DIO-NASH-vehicle = 5% TPGS/10% PVP-VA/85% 0.01M HCl), Compound A (Low, 60 mg/kg, BID) and the combination of Compound A (Low, 60 mg/kg, BID) with Compound B (10 mg/kg, QD) in the Gubra amylin NASH (GAN) mouse model.
**FIG. 8** shows PSR as percent area of liver tissue from subjects administered lean chow (wt), DIO-NASH-vehicle, Compound A, Compound B, the combination Compound A and Compound B, and CVC in the Gubra amylin NASH (GAN) mouse model using picrosirius red staining (PSR) from liver tissue. Data presented as mean ± SEM.
**FIG. 9** shows anti-smooth muscle antibody (aSMA) levels from subjects administered lean chow (wt), DIO-NASH-vehicle, Compound A, Compound B, the combination Compound A and Compound B, and CVC in the Gubra amylin NASH (GAN) mouse model. Data presented as mean ± SEM.
**FIG. 10** shows NAFLD activity score from subjects administered lean chow (wt), DIO-NASH-vehicle, Compound A, Compound B, the combination Compound A and Compound B, and CVC in the Gubra amylin NASH (GAN) mouse model.
**FIG. 11A****,** **FIG. 11B** and **FIG. 11C** show hepatocellular ballooning (**FIG. 11A**), lobular inflammation (**FIG. 11B**), and steatosis score (**FIG. 11C**) which were components used to determine the NAFLD activity score of **FIG. 10****.**
**FIG. 12** shows the effect of lean chow (wt), DIO-NASH-vehicle, Compound A, Compound B, the combination Compound A and Compound B, and CVC on liver total cholesterol levels in the Gubra amylin NASH (GAN) mouse model. Data presented as mean ± SEM.
**FIG. 13** shows a heatmap of liver cytokine levels measured by ELISA in GAN model. ****p < 0.0001, ***p < 0.001, **p < 0.01, *p < 0.05 vs. vehicle; one-way ANOVA, Tukey's post hoc test.

### Detailed Description

### Definitions and General Parameters

The following description sets forth exemplary methods, parameters and the like. It should be recognized, however, that such description is not intended as a limitation on the scope of the present disclosure but is instead provided as a description of exemplary embodiments.

As used in the present specification, the following words, phrases and symbols are generally intended to have the meanings as set forth below, except to the extent that the context in which they are used indicates otherwise.

As used herein generally, "COT inhibitor" or "compound which inhibits COT" means any therapeutic agent that reduces the activity of COT (cancer Osaka thyroid). Cancer Osaka thyroid (COT) is also known as "TPL2" (tumor progression locus), "MAP3K8" (mitogen-activated protein kinase kinase kinase 8) or "EST" (Ewing sarcoma transformant). Therefore, a "COT inhibitor" or "compound which inhibits COT" is also a "TPL2 inhibitor" or "TPL21".

As used herein generally, "ACC inhibitor" or "ACCi" means any therapeutic agent that reduces the activity of an acetyl CoA carboxylase enzyme.

The term "inhibition" indicates a decrease in the baseline activity of a biological activity or process. "Inhibition of activity of COT" or variants thereof refers to a decrease in activity in COT as a direct or indirect response to the presence of a compound of the present disclosure relative to the activity COT in the absence of the compound of the present disclosure. "Inhibition of COT" refers to a decrease in COT activity as a direct or indirect response to the presence of a compound described herein relative to the activity of COT in the absence of the compound described herein. In some embodiments, the inhibition of COT activity may be compared in the same subject prior to treatment, or other subjects not receiving the treatment.

"Treatment" or "treating" is an approach for obtaining beneficial or desired results including clinical results. Beneficial or desired clinical results may include one or more of the following: a) inhibiting the disease or condition (e.g., decreasing one or more symptoms resulting from the disease or condition, and/or diminishing the extent of the disease or condition); b) slowing or arresting the development of one or more clinical symptoms associated with the disease or condition (e.g., stabilizing the disease or condition, preventing or delaying the worsening or progression of the disease or condition, and/or preventing or delaying the spread (e.g., metastasis) of the disease or condition); and/or c) relieving the disease, that is, causing the regression of clinical symptoms (*e.g.*, ameliorating the disease state, providing partial or total remission of the disease or condition, enhancing effect of another medication, delaying the progression of the disease, increasing the quality of life, and/or prolonging survival.

"Prevention" or "preventing" means any treatment of a disease or condition that causes the clinical symptoms of the disease or condition not to develop. Compounds may, in some embodiments, be administered to a subject (including a human) who is at risk or has a family history of the disease or condition.

As used herein, the term "subject" refers to a mammalian subject. Exemplary subjects include humans, monkeys, dogs, cats, mice, rats, cows, horses, goats and sheep. In some embodiments, the subject is human. In some embodiments, the subject has a liver disease or condition and can be treated as described herein. In some embodiments, the subject has liver fibrosis. In some embodiments, the subject has NAFLD. In some embodiments, the human has stage 3 or stage 4 (F3 or F4) liver fibrosis. In some embodiments, the subject has NASH. In some embodiments, the subject has liver inflammation. In some embodiments, the subject has three or more features of metabolic syndrome (e.g., elevated blood pressure, abdominal obesity, elevated fasting plasma glucose, elevated liver lipids, elevated cholesterol and elevated collagen levels).

A "pharmaceutical composition" is intended to include the combination of an active agent with a carrier, inert or active, making the composition suitable for diagnostic or therapeutic use *in vitro, in vivo* or *ex vivo.*

The term "therapeutically effective amount" or "effective amount" of a compound described herein or a pharmaceutically acceptable salt, tautomer, stereoisomer, mixture of stereoisomers, or deuterated analog thereof means an amount sufficient to effect treatment when administered to a subject, to provide a therapeutic benefit such as amelioration of symptoms or slowing of disease progression. For example, a therapeutically effective amount may be an amount sufficient to decrease a symptom of a disease or condition responsive to inhibition of COT activity. The therapeutically effective amount may vary depending on the subject, and disease or condition being treated, the weight and age of the subject, the severity of the disease or condition, and the manner of administering, which can readily be determined by one or ordinary skill in the art.

A dash ("-") that is not between two letters or symbols is used to indicate a point of attachment for a substituent. For example, -C(O)NH₂ is attached through the carbon atom. A dash at the front or end of a chemical group is a matter of convenience; chemical groups may be depicted with or without one or more dashes without losing their ordinary meaning. A wavy line drawn through a line in a structure indicates a point of attachment of a group. Unless chemically or structurally required, no directionality is indicated or implied by the order in which a chemical group is written or named.

The prefix "Cᵤ₋ᵥ" indicates that the following group has from u to v carbon atoms. For example, "C₁₋₆ alkyl" indicates that the alkyl group has from 1 to 6 carbon atoms.

All numerical designations, e.g., pH, temperature, time, concentration, and molecular weight, including ranges, are approximations which are varied ( + ) or ( - ) by increments of 0.1 or 10%. It also is to be understood, although not always explicitly stated, that the reagents described herein are merely exemplary and that equivalents of such are known in the art. Also, the singular forms "a" and "the" include plural references unless the context clearly dictates otherwise. Thus, e.g., reference to "the compound" includes a plurality of such compounds and reference to "the assay" includes reference to one or more assays and equivalents thereof known to those skilled in the art.

"Alkyl" refers to an unbranched or branched saturated hydrocarbon chain. As used herein, alkyl has 1 to 20 carbon atoms (*i.e.,* C₁₋₂₀ alkyl), 1 to 8 carbon atoms (*i.e.,* C₁₋₈ alkyl), 1 to 6 carbon atoms (*i.e.,* C₁₋₆ alkyl), or 1 to 4 carbon atoms (*i.e.,* C₁₋₄ alkyl). Examples of alkyl groups include methyl, ethyl, propyl, isopropyl, n-butyl, sec-butyl, iso-butyl, tert-butyl, pentyl, 2-pentyl, isopentyl, neopentyl, hexyl, 2-hexyl, 3-hexyl, and 3-methylpentyl. When an alkyl residue having a specific number of carbons is named by chemical name or identified by molecular formula, all positional isomers having that number of carbons may be encompassed; thus, for example, "butyl" includes n-butyl (i.e., -(CH₂)₃CH₃), sec-butyl (i.e., -CH(CH₃)CH₂CH₃), isobutyl (i.e., -CH₂CH(CH₃)₂) and tert-butyl (i.e., -C(CH₃)₃); and "propyl" includes n-propyl (i.e., -(CH₂)₂CH₃) and isopropyl (i.e., -CH(CH₃)₂).

"Alkenyl" refers to an alkyl group containing at least one carbon-carbon double bond and having from 2 to 20 carbon atoms (*i.e.,* C₂₋₂₀ alkenyl), 2 to 8 carbon atoms (*i.e.,* C₂₋₈ alkenyl), 2 to 6 carbon atoms (*i.e.,* C₂₋₆ alkenyl), or 2 to 4 carbon atoms (*i.e.,* C₂₋₄ alkenyl). Examples of alkenyl groups include ethenyl, propenyl, butadienyl (including 1,2-butadienyl and 1,3-butadienyl).

"Alkynyl" refers to an alkyl group containing at least one carbon-carbon triple bond and having from 2 to 20 carbon atoms (*i.e.,* C₂₋₂₀ alkynyl), 2 to 8 carbon atoms (*i.e.,* C₂₋₈ alkynyl), 2 to 6 carbon atoms (*i.e.,* C₂₋₆ alkynyl), or 2 to 4 carbon atoms (*i.e.,* C₂₋₄ alkynyl). The term "alkynyl" also includes those groups having one triple bond and one double bond.

"Alkoxy" refers to the group "alkyl-O-". Examples of alkoxy groups include methoxy, ethoxy, n-propoxy, iso-propoxy, n-butoxy, tert-butoxy, sec-butoxy, n-pentoxy, n-hexoxy, and 1,2-dimethylbutoxy.

"Haloalkoxy" refers to an alkoxy group as defined above, wherein one or more hydrogen atoms are replaced by a halogen.

"Alkylthio" refers to the group "alkyl-S-".

"Acyl" refers to a group -C(O)R, wherein R is hydrogen, alkyl, cycloalkyl, heterocyclyl, aryl, heteroalkyl, or heteroaryl; each of which may be optionally substituted, as defined herein. Examples of acyl include formyl, acetyl, cylcohexylcarbonyl, cyclohexylmethyl-carbonyl, and benzoyl.

"Amido" refers to both a "C-amido" group which refers to the group -C(O)NR^{y}R^{z} and an "N-amido" group which refers to the group -NR^{y}C(O)R^{z}, wherein R^{y} and R^{z} are independently selected from the group consisting of hydrogen, alkyl, aryl, haloalkyl, or heteroaryl; each of which may be optionally substituted.

"Amino" refers to the group -NR^{y}R^{z} wherein R^{y} and R^{z} are independently selected from the group consisting of hydrogen, alkyl, haloalkyl, aryl, or heteroaryl; each of which may be optionally substituted.

"Amidino" refers to -C(NH)(NH₂).

"Aryl" refers to an aromatic carbocyclic group having a single ring (e.g., monocyclic) or multiple rings (e.g., bicyclic or tricyclic) including fused systems. As used herein, aryl has 6 to 20 ring carbon atoms (*i.e.,* C₆₋₂₀ aryl), 6 to 12 carbon ring atoms (*i.e.,* C₆₋₁₂ aryl), or 6 to 10 carbon ring atoms (*i.e.,* C₆₋₁₀ aryl). Examples of aryl groups include phenyl, naphthylenyl, fluorenyl, and anthracenyl. Aryl, however, does not encompass or overlap in any way with heteroaryl defined below. If one or more aryl groups are fused with a heteroaryl, the resulting ring system is heteroaryl. If one or more aryl groups are fused with a heterocyclyl, the resulting ring system is heterocyclyl.

"Azido" refers to -N₃.

"Carbamoyl" refers to both an "O-carbamoyl" group which refers to the group -O-C(O)NR^{y}R^{z} and an "N-carbamoyl" group which refers to the group -NR^{y}C(O)OR^{z}, wherein R^{y} and R^{z} are independently selected from the group consisting of hydrogen, alkyl, aryl, haloalkyl, or heteroaryl; each of which may be optionally substituted.

"Carboxyl" refers to -C(O)OH.

"Carboxyl ester" refers to both -OC(O)R and -C(O)OR, wherein R is hydrogen, alkyl, cycloalkyl, heterocyclyl, aryl, heteroalkyl, or heteroaryl; each of which may be optionally substituted, as defined herein.

"Cyano" or "carbonitrile" refers to the group -CN.

"Cycloalkyl" refers to a saturated or partially unsaturated cyclic alkyl group having a single ring or multiple rings including fused, bridged, and spiro ring systems. The term "cycloalkyl" includes cycloalkenyl groups (i.e., the cyclic group having at least one double bond). As used herein, cycloalkyl has from 3 to 20 ring carbon atoms (*i.e.,* C₃₋₂₀ cycloalkyl), 3 to 12 ring carbon atoms (*i.e.,* C₃₋₁₂ cycloalkyl), 3 to 10 ring carbon atoms (*i.e.,* C₃₋₁₀ cycloalkyl), 3 to 8 ring carbon atoms (*i.e.,* C₃₋₈ cycloalkyl), or 3 to 6 ring carbon atoms (*i.e.,* C₃₋₆ cycloalkyl). Examples of cycloalkyl groups include cyclopropyl, cyclobutyl, cyclopentyl, and cyclohexyl.

"Guanidino" refers to -NHC(NH)(NH₂).

"Hydrazino" refers to -NHNH₂.

"Imino" refers to a group -C(NR)R, wherein each R is alkyl, cycloalkyl, heterocyclyl, aryl, heteroalkyl, or heteroaryl; each of which may be optionally substituted, as defined herein.

"Halogen" or "halo" includes fluoro, chloro, bromo, and iodo. "Haloalkyl" refers to an unbranched or branched alkyl group as defined above, wherein one or more hydrogen atoms are replaced by a halogen. For example, where a residue is substituted with more than one halogen, it may be referred to by using a prefix corresponding to the number of halogen moieties attached. Dihaloalkyl and trihaloalkyl refer to alkyl substituted with two ("di") or three ("tri") halo groups, which may be, but are not necessarily, the same halogen. Examples of haloalkyl include difluoromethyl (-CHF₂) and trifluoromethyl (-CF₃).

"Heteroalkyl" refers to an alkyl group in which one or more of the carbon atoms (and any associated hydrogen atoms) are each independently replaced with the same or different heteroatomic group. The term "heteroalkyl" includes unbranched or branched saturated chains having carbon and heteroatoms. By way of example, 1, 2 or 3 carbon atoms may be independently replaced with the same or different heteroatomic group. Heteroatomic groups include -NR-, -O-, -S-,
-S(O)-, and -S(O)₂-, where R is H, alkyl, aryl, cycloalkyl, heteroalkyl, heteroaryl or heterocyclyl, each of which may be optionally substituted. Examples of heteroalkyl groups include -OCH₃,
-CH₂OCH₃, -SCH₃, -CH₂SCH₃, -NRCH₃, and -CH₂NRCH₃, where R is hydrogen, alkyl, aryl, arylalkyl, heteroalkyl, or heteroaryl, each of which may be optionally substituted. As used herein, heteroalkyl include 1 to 10 carbon atoms, 1 to 8 carbon atoms, or 1 to 4 carbon atoms; and 1 to 3 heteroatoms, 1 to 2 heteroatoms, or 1 heteroatom.

"Heteroaryl" refers to an aromatic group having a single ring, multiple rings, or multiple fused rings, with one or more ring heteroatoms independently selected from nitrogen, oxygen, and sulfur. As used herein, heteroaryl includes 1 to 20 ring carbon atoms (*i.e.,* C₁₋₂₀ heteroaryl), 3 to 12 ring carbon atoms (*i.e.,* C₃₋₁₂ heteroaryl), or 3 to 8 carbon ring atoms (*i.e.,* C₃₋₈ heteroaryl); and 1 to 5 heteroatoms, 1 to 4 heteroatoms, 1 to 3 ring heteroatoms, 1 to 2 ring heteroatoms, or 1 ring heteroatom independently selected from nitrogen, oxygen, and sulfur. Examples of heteroaryl groups include pyrimidinyl, purinyl, pyridyl, pyridazinyl, benzothiazolyl, and pyrazolyl. Examples of the fused-heteroaryl rings include benzo[d]thiazolyl, quinolinyl, isoquinolinyl, benzo[b]thiophenyl, indazolyl, benzo[d]imidazolyl, pyrazolo[1,5-a]pyridinyl, and imidazo[1,5-a]pyridinyl, where the heteroaryl can be bound via either ring of the fused system. Any aromatic ring, having a single ring or multiple fused rings, containing at least one heteroatom, is considered a heteroaryl regardless of the attachment to the remainder of the molecule (i.e., through any one of the fused rings). Heteroaryl does not encompass or overlap with aryl as defined above.

"Heterocyclyl" refers to a saturated or unsaturated cyclic alkyl group, with one or more ring heteroatoms independently selected from nitrogen, oxygen and sulfur. The term "heterocyclyl" includes heterocycloalkenyl groups (i.e., the heterocyclyl group having at least one double bond), bridged-heterocyclyl groups, fused-heterocyclyl groups, and spiro-heterocyclyl groups. A heterocyclyl may be a single ring or multiple rings wherein the multiple rings may be fused, bridged, or spiro. Any non-aromatic ring containing at least one heteroatom is considered a heterocyclyl, regardless of the attachment (i.e., can be bound through a carbon atom or a heteroatom). Further, the term heterocyclyl is intended to encompass any non-aromatic ring containing at least one heteroatom, which ring may be fused to an aryl or heteroaryl ring, regardless of the attachment to the remainder of the molecule. As used herein, heterocyclyl has 2 to 20 ring carbon atoms (*i.e.*, C₂₋₂₀ heterocyclyl), 2 to 12 ring carbon atoms (*i.e.,* C₂₋₁₂ heterocyclyl), 2 to 10 ring carbon atoms (*i.e.,* C₂₋₁₀ heterocyclyl), 2 to 8 ring carbon atoms (*i.e.,* C₂₋₈ heterocyclyl), 3 to 12 ring carbon atoms (*i.e.,* C₃₋₁₂ heterocyclyl), 3 to 8 ring carbon atoms (*i.e.,* C₃₋₈ heterocyclyl), or 3 to 6 ring carbon atoms (*i.e.,* C₃₋₆ heterocyclyl); having 1 to 5 ring heteroatoms, 1 to 4 ring heteroatoms, 1 to 3 ring heteroatoms, 1 to 2 ring heteroatoms, or 1 ring heteroatom independently selected from nitrogen, sulfur or oxygen. A heterocyclyl may contain one or more oxo and/or thioxo groups. Examples of heterocyclyl groups include pyrrolidinyl, piperidinyl, piperazinyl, oxetanyl, dioxolanyl, azetidinyl, and morpholinyl. As used herein, the term "bridged-heterocyclyl" refers to a four- to ten-membered cyclic moiety connected at two non-adjacent atoms of the heterocyclyl with one or more (e.g., 1 or 2) four- to ten-membered cyclic moiety having at least one heteroatom where each heteroatom is independently selected from nitrogen, oxygen, and sulfur. As used herein, bridged- heterocyclyl includes bicyclic and tricyclic ring systems. Also used herein, the term "spiro-heterocyclyl" refers to a ring system in which a three- to ten-membered heterocyclyl has one or more additional ring, wherein the one or more additional ring is three- to ten-membered cycloalkyl or three- to ten-membered heterocyclyl, where a single atom of the one or more additional ring is also an atom of the three- to ten-membered heterocyclyl. Examples of the spiro-heterocyclyl rings include bicyclic and tricyclic ring systems, such as 2-oxa-7-azaspiro[3.5]nonanyl, 2-oxa-6-azaspiro[3.4]octanyl, and 6-oxa-1-azaspiro[3.3]heptanyl. Examples of the fused-heterocyclyl rings include 1,2,3,4-tetrahydroisoquinolinyl, 1-oxo-1,2,3,4-tetrahydroisoquinolinyl, 1-oxo-1,2-dihydroisoquinolinyl, 4,5,6,7-tetrahydrothieno[2,3-c]pyridinyl, indolinyl, and isoindolinyl, where the heterocyclyl can be bound via either ring of the fused system.

"Hydroxy" or "hydroxyl" refers to the group -OH. "Hydroxyalkyl" refers to an unbranched or branched alkyl group as defined above, wherein one or more hydrogen atoms are replaced by a hydroxyl.

"Oxo" refers to the group (=O) or (O).

"Nitro" refers to the group -NO₂.

"Sulfonyl" refers to the group -S(O)₂R, where R is alkyl, haloalkyl, heterocyclyl, cycloalkyl, heteroaryl, or aryl. Examples of sulfonyl are methylsulfonyl, ethylsulfonyl, phenylsulfonyl, and toluene sulfonyl.

"Alkylsulfonyl" refers to the group -S(O)₂R, where R is alkyl.

"Alkylsulfinyl" refers to the group -S(O)R, where R is alkyl.

"Thiocyanate" -SCN.

"Thiol" refers to the group -SR, where R is alkyl, haloalkyl, heterocyclyl, cycloalkyl, heteroaryl, or aryl.

"Thioxo" or "thione" refer to the group (=S) or (S).

Certain commonly used alternative chemical names may be used. For example, a divalent group such as a divalent "alkyl" group, a divalent "aryl" group, etc., may also be referred to as an "alkylene" group or an "alkylenyl" group, an "arylene" group or an "arylenyl" group, respectively. Also, unless indicated explicitly otherwise, where combinations of groups are referred to herein as one moiety, e.g., arylalkyl, the last-mentioned group contains the atom by which the moiety is attached to the rest of the molecule.

The terms "optional" or "optionally" means that the subsequently described event or circumstance may or may not occur, and that the description includes instances where said event or circumstance occurs and instances in which it does not. Also, the term "optionally substituted" refers to any one or more hydrogen atoms on the designated atom or group may or may not be replaced by a moiety other than hydrogen.

Some of the compounds exist as tautomers. Tautomers are in equilibrium with one another. For example, amide containing compounds may exist in equilibrium with imidic acid tautomers. Regardless of which tautomer is shown, and regardless of the nature of the equilibrium among tautomers, the compounds are understood by one of ordinary skill in the art to comprise both amide and imidic acid tautomers. Thus, the amide containing compounds are understood to include their imidic acid tautomers. Likewise, the imidic acid containing compounds are understood to include their amide tautomers.

Any formula or structure given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, phosphorus, fluorine and chlorine, such as ²H (deuterium, D), ³H (tritium), ¹¹C, ¹³C, ¹⁴C, ¹⁵N, ¹⁸F, ³¹P, ³²P, ³⁵S, ³⁶Cl and ¹²⁵I. Various isotopically labeled compounds of the present disclosure, for example those into which radioactive isotopes such as ³H, ¹³C and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients.

The disclosure also includes "deuterated analogues" of compounds of Formula I in which from 1 to n hydrogens attached to a carbon atom is/are replaced by deuterium, in which n is the number of hydrogens in the molecule. Such compounds exhibit increased resistance to metabolism and are thus useful for increasing the half-life of any compound of Formula I when administered to a mammal, such as a human. See, for example, Foster, "Deuterium Isotope Effects in Studies of Drug Metabolism," Trends Pharmacol. Sci. 5(12):524-527 (1984). Such compounds are synthesized by means well known in the art, for example by employing starting materials in which one or more hydrogens have been replaced by deuterium.

Deuterium labelled or substituted therapeutic compounds of the disclosure may have improved DMPK (drug metabolism and pharmacokinetics) properties, relating to absorption, distribution, metabolism and excretion (ADME). Substitution with heavier isotopes such as deuterium may afford certain therapeutic advantages resulting from greater metabolic stability, for example increased *in vivo* half-life, reduced dosage requirements and/or an improvement in therapeutic index. An ¹⁸F labeled compound may be useful for PET or SPECT studies. Isotopically labeled compounds of this disclosure and prodrugs thereof can generally be prepared by carrying out the procedures disclosed in the schemes or in the examples and preparations described below by substituting a readily available isotopically labeled reagent for a non-isotopically labeled reagent. It is understood that deuterium in this context is regarded as a substituent in the compound of Formula I.

The concentration of such a heavier isotope, specifically deuterium, may be defined by an isotopic enrichment factor. In the compounds of this disclosure any atom not specifically designated as a certain isotope is meant to represent any stable isotope of that atom. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium.

In some cases, the compounds of this disclosure are capable of forming acid and/or base salts by virtue of the presence of amino and/or carboxyl groups or groups similar thereto.

Provided are also pharmaceutically acceptable salts, hydrates, solvates, tautomeric forms, and polymorphs of the compounds described herein. "Pharmaceutically acceptable" or "physiologically acceptable" refer to compounds, salts, compositions, dosage forms and other materials which are useful in preparing a pharmaceutical composition that is suitable for veterinary or human pharmaceutical use.

The term "pharmaceutically acceptable salt" of a given compound refers to salts that retain the biological effectiveness and properties of the given compound, and which are not biologically or otherwise undesirable. "Pharmaceutically acceptable salts" or "physiologically acceptable salts" include, for example, salts with inorganic acids and salts with an organic acid. In addition, if the compounds described herein are obtained as an acid addition salt, the free base can be obtained by basifying a solution of the acid salt. Conversely, if the product is a free base, an addition salt, such as a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare nontoxic pharmaceutically acceptable addition salts. Pharmaceutically acceptable acid addition salts may be prepared from inorganic and organic acids. Salts derived from inorganic acids include hydrochloric acid, hydrobromic acid, sulfuric acid, nitric acid, and phosphoric acid. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, and salicylic acid. Likewise, pharmaceutically acceptable base addition salts can be prepared from inorganic and organic bases. Salts derived from inorganic bases include, by way of example only, sodium, potassium, lithium, ammonium, calcium and magnesium salts. Salts derived from organic bases include salts of primary, secondary and tertiary amines, such as alkyl amines (i.e., NH₂(alkyl)), dialkyl amines (i.e., HN(alkyl)₂), trialkyl amines (i.e., N(alkyl)₃), substituted alkyl amines (i.e., NH₂(substituted alkyl)), di(substituted alkyl) amines (i.e., HN(substituted alkyl)₂), tri(substituted alkyl) amines (i.e., N(substituted alkyl)₃), alkenyl amines (i.e., NH₂(alkenyl)), dialkenyl amines (i.e., HN(alkenyl)₂), trialkenyl amines (i.e., N(alkenyl)₃), substituted alkenyl amines (i.e., NH₂(substituted alkenyl)), di(substituted alkenyl) amines (i.e., HN(substituted alkenyl)₂), tri(substituted alkenyl) amines (i.e., N(substituted alkenyl)₃, mono-, di- or tri- cycloalkyl amines (i.e., NH₂(cycloalkyl), HN(cycloalkyl)₂, N(cycloalkyl)₃), mono-, di- or tri- arylamines (i.e., NH₂(aryl), HN(aryl)₂, N(aryl)₃), or mixed amines, etc. Specific examples of suitable amines include, by way of example only, isopropylamine, trimethyl amine, diethyl amine, tri(iso-propyl) amine, tri(n-propyl) amine, ethanolamine, 2-dimethylaminoethanol, piperazine, piperidine, morpholine, and N-ethylpiperidine.

The term "substituted" means that any one or more hydrogen atoms on the designated atom or group is replaced with one or more substituents other than hydrogen, provided that the designated atom's normal valence is not exceeded. The one or more substituents include alkyl, alkenyl, alkynyl, alkoxy, acyl, amino, amido, amidino, aryl, azido, carbamoyl, carboxyl, carboxyl ester, cyano, guanidino, halo, haloalkyl, haloalkoxy, heteroalkyl, heteroaryl, heterocyclyl, hydroxy, hydrazino, imino, oxo, nitro, alkylsulfinyl, sulfonic acid, alkylsulfonyl, thiocyanate, thiol, thione, or combinations thereof. Polymers or similar indefinite structures arrived at by defining substituents with further substituents appended ad infinitum (e.g., a substituted aryl having a substituted alkyl which is itself substituted with a substituted aryl group, which is further substituted by a substituted heteroalkyl group, etc.) are not intended for inclusion herein. Unless otherwise noted, the maximum number of serial substitutions in compounds described herein is three. For example, serial substitutions of substituted aryl groups with two other substituted aryl groups are limited to ((substituted aryl)substituted aryl) substituted aryl. Similarly, the above definitions are not intended to include impermissible substitution patterns (e.g., methyl substituted with 5 fluorines or heteroaryl groups having two adjacent oxygen ring atoms). Such impermissible substitution patterns are well known to the skilled artisan. When used to modify a chemical group, the term "substituted" may describe other chemical groups defined herein. Unless specified otherwise, where a group is described as optionally substituted, any substituents of the group are themselves unsubstituted. For example, in some embodiments, the term "substituted alkyl" refers to an alkyl group having one or more substituents including hydroxyl, halo, alkoxy, cycloalkyl, heterocyclyl, aryl, and heteroaryl. In other embodiments, the one or more substituents may be further substituted with halo, alkyl, haloalkyl, hydroxyl, alkoxy, cycloalkyl, heterocyclyl, aryl, or heteroaryl, each of which is substituted. In other embodiments, the substituents may be further substituted with halo, alkyl, haloalkyl, alkoxy, hydroxyl, cycloalkyl, heterocyclyl, aryl, or heteroaryl, each of which is unsubstituted.

A "solvate" is formed by the interaction of a solvent and a compound. Solvates of salts of the compounds described herein are also provided. Hydrates of the compounds described herein are also provided.

### Methods

It has been shown that both obesity and insulin resistance play a role in the disease process of NAFLD. In addition to a poor diet, NAFLD has several other known causes. For example, NAFLD can be caused by certain medications, such as amiodarone, antiviral drugs (e.g., nucleoside analogues), aspirin (rarely as part of Reye's syndrome in children), corticosteroids, methotrexate, tamoxifen, or tetracycline. NAFLD is associated with insulin resistance and metabolic syndrome (obesity, combined hyperlipidemia, diabetes mellitus (type II) and high blood pressure). NAFLD is considered to cover a spectrum of disease activity, and begins as fatty accumulation in the liver (hepatic steatosis). NAFLD has also been linked to the consumption of soft drinks through the presence of high fructose corn syrup which may cause increased deposition of fat in the abdomen, although the consumption of sucrose shows a similar effect (likely due to its breakdown into fructose). Genetics has also been known to play a role, as two genetic mutations for this susceptibility have been identified. If left untreated, NAFLD can develop into non-alcoholic steatohepatitis (NASH), which is the most extreme form of NAFLD, a state in which steatosis is combined with inflammation and fibrosis.

As shown herein, COT (or TPL2) inhibition reduces fibrosis in a choline-deficient high-fat diet (CDHFD) animal models. Described herein is a method of treating, stabilizing, or lessening the severity or progression of nonalcoholic steatohepatitis (NASH) in a subject in need thereof, comprising administering to the subject a therapeutically effective amount of a compound that inhibits COT. In certain embodiments, the treating comprises inhibiting the progression of fibrosis as a result of NASH. Also described herein is a method of treating, stabilizing, or lessening the severity or progression of liver fibrosis in a subject in need thereof, comprising administering to the subject a therapeutically effective amount of a compound that inhibits COT. Liver fibrosis is the excessive accumulation of extracellular matrix proteins, including collagen. Liver fibrosis frequently occurs various types of chronic liver diseases. In certain embodiments, advanced liver fibrosis results in cirrhosis and liver failure. In some embodiments, COT inhibition reduces glycolysis in M1 macrophages and IL-1β secretion. In some embodiments, COT inhibition increases M2 macrophage ability to clear apoptotic cells.

NASH is regarded as a major cause of cirrhosis of the liver. Described herein is a method for treating, stabilizing, or lessening the severity or progression of cirrhosis of the liver, the method comprising administering to a subject in need thereof, an effective amount of a compound which inhibits COT. Also described herein is a method for reducing liver cirrhosis, the method comprising administering to a subject in need thereof, an effective amount of a compound which inhibits COT. In one embodiment, cirrhosis is characterized pathologically by loss of the normal microscopic lobular architecture, with fibrosis and nodular regeneration. Methods for measuring the extent of cirrhosis are well known in the art. In one embodiment, the level of cirrhosis is reduced by 5% to 100%. In one embodiment, the level of cirrhosis is reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100% in the subject.

Methods for measuring liver histologies in a NASH subject, such as changes in the extent of fibrosis, lobular hepatitis, and periportal bridging necrosis, are well known in the art. Liver disease in a NASH subject can be classified into 4 stages: F0 indicates no fibrosis; F1 indicates mild fibrosis; F2 indicates moderate fibrosis; F3 indicates severe fibrosis; and F4 indicates cirrhosis. Fibrosis Score refers to a scoring system for fibrosis as described by Kleiner et al. (Hepatology, Design and validation of a histological scoring system for nonalcoholic fatty liver disease (2005), 41: 1313-1321). In one embodiment, treatment as described herein may improve a in a NASH subject's fibrosis from baseline, for example, improving from F4 to F3, F3 to F2, or F2 to F1. In certain embodiments, the fibrosis is F1 fibrosis. In certain embodiments, the fibrosis is F2 fibrosis. In certain embodiments, the fibrosis is F3 fibrosis. In certain embodiments, the fibrosis is F4 fibrosis. In certain embodiments, the fibrosis is F3-F4 fibrosis.

In one embodiment, a NASH subject's fibrosis score is improved by one or more following 24 weeks of daily treatment. Described is a method for treating, stabilizing, or lessening the severity or progression of liver fibrosis in a subject in need thereof, wherein the liver fibrosis stage of the subject is F3, comprising administering to the subject a therapeutically effective amount of a COT inhibitor, optionally in combination with a therapeutically effective amount of an ACC inhibitor. Described herein is a method for treating, stabilizing, or lessening the severity or progression of non-alcoholic steatohepatitis (NASH) in a subject, the method comprising administering to a subject in need thereof, an effective amount of a compound which inhibits TPL2, or COT, optionally in combination with a therapeutically effective amount of an ACC inhibitor. Also described is a method for treating, stabilizing, or lessening the severity or progression of liver fibrosis in a subject in need thereof, wherein the liver fibrosis stage of the subject is F4, comprising administering to the subject a therapeutically effective amount of a COT inhibitor, optionally in combination with a therapeutically effective amount of an ACC inhibitor.

In one embodiment, the level of liver fibrosis, which is the formation of fibrous tissue, fibroid or fibrous degeneration, is reduced by more than 90%. In one embodiment, the level of fibrosis, which is the formation of fibrous tissue, fibroid or fibrous degeneration, is reduced by at least 90%, at least 80%, at least 70%, at least 60%, at least 50%, at least 40%, at least 30%, at least 20%, at least 10%, at least 5% or at least 2%.

Described herein are methods for reducing the level of fibrogenesis in the liver. Liver fibrogenesis is the process leading to the deposition of an excess of extracellular matrix components in the liver known as fibrosis. In one embodiment, the level of fibrogenesis is reduced by more than 90%. In one embodiment, the level of fibrogenesis is reduced by at least 90%, at least 80%, at least 70%, at least 60%, at least 50%, at least 40%, at least 30%, at least 20%, at least 10%, at least 5% or at least 2%.

Described herein are methods for reducing the level of liver stiffness. Liver fibrogenesis is the process leading to the deposition of an excess of extracellular matrix components in the liver known as fibrosis. In one embodiment, the level of liver stiffness is reduced by more than 90%. In one embodiment, the level of liver stiffness is reduced by at least 90%, at least 80%, at least 70%, at least 60%, at least 50%, at least 40%, at least 30%, at least 20%, at least 10%, at least 5% or at least 2%.

Described herein are methods for reducing liver hydroxyproline levels in a subject, the method comprising administering to a subject in need thereof, an effective amount of a compound which inhibits COT. In one embodiment, the liver hydroxyproline level is reduced by more than 90%. In one embodiment, the liver hydroxyproline level is reduced by at least 90%, at least 80%, at least 70%, at least 60%, at least 50%, at least 40%, at least 30%, at least 20%, at least 10%, at least 5% or at least 2%.

Described herein are methods for reducing hepatic collagen production in a subject, the method comprising administering to a subject in need thereof, an effective amount of a compound which inhibits COT. In one embodiment, hepatic collagen production is reduced by more than 90%. In one embodiment, hepatic collagen production is reduced by at least 90%, at least 80%, at least 70%, at least 60%, at least 50%, at least 40%, at least 30%, at least 20%, at least 10%, at least 5% or at least 2%.

Described herein are methods for reducing one or more of body weight, fat mass, liver cholesterol, or liver lipids in a subject, the method comprising administering to a subject in need thereof, an effective amount of a compound which inhibits COT. In one embodiment, one or more of body weight, fat mass, liver cholesterol, or liver lipids is reduced by more than 90%. In one embodiment, one or more of body weight, fat mass, liver cholesterol, or liver lipids is reduced by at least 90%, at least 80%, at least 70%, at least 60%, at least 50%, at least 40%, at least 30%, at least 20%, at least 10%, at least 5% or at least 2%.

Described herein are methods for treating, stabilizing, or lessening the severity or progression of one or more of nonalcoholic steatohepatitis (NASH), nonalcoholic fatty liver disease (NAFLD), cirrhosis of the liver, liver fibrosis, liver fibrogenesis, liver stiffness, or reducing one or more of immune cell infiltration, cytokine production in a liver, liver hydroxyproline levels, hepatic collagen production, body weight, fat mass, liver cholesterol, or liver lipids, in a subject in need thereof, comprising administering to the subject a therapeutically effective amount of a compound which inhibits COT.

Described herein are methods for treating, stabilizing, or lessening the severity or progression of nonalcoholic steatohepatitis (NASH) in a subject in need thereof, comprising administering to the subject a therapeutically effective amount of a compound which inhibits COT, optionally in combination with an ACC inhibitor.

Described herein are methods for treating, stabilizing, or lessening the severity or progression of nonalcoholic fatty liver disease (NAFLD) in a subject in need thereof, comprising administering to the subject a therapeutically effective amount of a compound which inhibits COT, optionally in combination with an ACC inhibitor.

Described herein are methods for treating, stabilizing, or lessening the severity or progression of cirrhosis of the liver in a subject in need thereof, comprising administering to the subject a therapeutically effective amount of a compound which inhibits COT, optionally in combination with an ACC inhibitor.

Described herein are methods for treating, stabilizing, or lessening the severity or progression of liver fibrosis in a subject in need thereof, comprising administering to the subject a therapeutically effective amount of a compound which inhibits COT, optionally in combination with an ACC inhibitor.

Described herein are methods for treating, stabilizing, or lessening the severity or progression of liver fibrogenesis in a subject in need thereof, comprising administering to the subject a therapeutically effective amount of a compound which inhibits COT, optionally in combination with an ACC inhibitor.

Described herein are methods for treating, stabilizing, or lessening the severity or progression of liver stiffness in a subject in need thereof, comprising administering to the subject a therapeutically effective amount of a compound which inhibits COT, optionally in combination with an ACC inhibitor.

Described herein is a method for reducing glycolysis in M1 macrophages and IL-1β secretion comprising administering to a subject in need thereof an effective amount of a compound which inhibits COT.

Described herein is a method for increasing M2 macrophage ability to clear apoptotic cells, comprising administering to a subject in need thereof an effective amount of a compound which inhibits COT.

Described herein are methods for reducing immune cell infiltration in a liver of a subject in need thereof, comprising administering to the subject a therapeutically effective amount of a compound which inhibits COT, optionally in combination with an ACC inhibitor. In certain embodiments, described is a method for reducing immune cell infiltration in the liver of a subject, the method comprising administering to a subject in need thereof, an effective amount of a compound which inhibits COT.

Described herein are methods for reducing cytokine production in a liver of a subject in need thereof, comprising administering to the subject a therapeutically effective amount of a compound which inhibits COT, optionally in combination with an ACC inhibitor.

Described herein are methods for reducing liver hydroxyproline levels in a subject in need thereof, comprising administering to the subject a therapeutically effective amount of a compound which inhibits COT, optionally in combination with an ACC inhibitor.

Described herein are methods for reducing hepatic collagen production in a subject in need thereof, comprising administering to the subject a therapeutically effective amount of a compound which inhibits COT, optionally in combination with an ACC inhibitor.

Described herein are methods for reducing body weight in a subject in need thereof, comprising administering to the subject a therapeutically effective amount of a compound which inhibits COT, optionally in combination with an ACC inhibitor.

Described herein are methods for reducing fat mass in a subject in need thereof, comprising administering to the subject a therapeutically effective amount of a compound which inhibits COT, optionally in combination with an ACC inhibitor.

Described herein are methods for reducing liver cholesterol in a subject in need thereof, comprising administering to the subject a therapeutically effective amount of a compound which inhibits COT, optionally in combination with an ACC inhibitor.

Described herein are methods for reducing hepatic ballooning in a subject in need thereof, comprising administering to the subject a therapeutically effective amount of a compound which inhibits COT, optionally in combination with an ACC inhibitor.

Described herein are methods for reducing liver lipids in a subject in need thereof, comprising administering to the subject a therapeutically effective amount of a compound which inhibits COT, optionally in combination with an ACC inhibitor.

Described herein are methods for reducing liver inflammation in a subject in need thereof, comprising administering to the subject a therapeutically effective amount of a compound which inhibits COT, optionally in combination with an ACC inhibitor.

Described herein is a method for reducing cytokine production in a liver, the method comprising administering to a subject in need thereof, an effective amount of a compound which inhibits COT.

Described herein is a method for reducing glycolysis in M1 macrophages and IL-1β secretion comprising administering to a subject in need thereof an effective amount of a compound which inhibits COT.

Described herein is a method for increasing M2 macrophage ability to clear apoptotic cells, comprising administering to a subject in need thereof an effective amount of a compound which inhibits COT.

Exemplary COT inhibitors for use in the methods described herein are as follows. Specific COT inhibitors as well as methods for preparing COT inhibitors as described above can be found in WO2020252151A1, WO2018005435A1, WO2017007689A1, and WO2017007694A1.

The compound that inhibits COT may be a compound of Formula I: 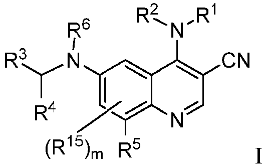 or a pharmaceutically acceptable salt, stereoisomer, mixture of stereoisomers, or deuterated analog thereof, wherein:
R¹ is hydrogen, -O-R⁷, -N(R⁸)(R⁹), -C(O)-R⁷, -S(O)₂-R⁷, -C₁₋₉ alkyl, C₂₋₆ alkenyl, C₂₋₆ alkynyl, C₃₋₁₅ cycloalkyl, heterocyclyl, aryl, or heteroaryl; wherein each C₁₋₉ alkyl, C₂₋₆ alkenyl, C₂₋₆ alkynyl, C₃₋₁₅ cycloalkyl, heterocyclyl, aryl, and heteroaryl may be optionally substituted with one to four Z¹;
R² is hydrogen, -C(O)-R⁷, -C(O)O-R⁷, -C(O)N(R⁷)₂, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl may be optionally substituted with one to four Z²;
or R¹ and R² together with the nitrogen to which they are attached to form a heterocyclyl or heteroaryl, wherein each heterocyclyl or heteroaryl is optionally substituted with one to four Z²;
R³ is heterocyclyl or heteroaryl, wherein each heterocyclyl or heteroaryl is optionally substituted with one to four Z³;
R⁴ is aryl, heterocyclyl, or heteroaryl, wherein each aryl, heterocyclyl, or heteroaryl is optionally substituted with one to four Z⁴;
R⁵ is hydrogen, halo, -CN, -NO₂, -O-R⁷, -N(R⁸)(R⁹), -S(O)R⁷, -S(O)₂R⁷, -S(O)₂N(R⁷)₂, -C(O)R⁷, -OC(O)-R⁷, -C(O)O-R⁷, -OC(O)O-R⁷, -OC(O)N(R¹⁰)(R¹¹), -C(O)N(R⁷)₂, -N(R⁷)C(O)(R⁷), C₁₋₉ alkyl, C₂₋₆ alkenyl, C₂₋₆ alkynyl, C₁₋₉ alkylthio, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₉ alkylthio, C₁₋₆ haloalkyl, C₃₋₁₅ cycloalkyl, aryl, heterocyclyl, and heteroaryl may be optionally substituted with one to four Z⁵;
R⁶ is hydrogen, -C(O)-R⁷, -C(O)O-R⁷, -C(O)N(R⁷)₂, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl may be optionally substituted with one to four Z⁶;
each R⁷ is independently hydrogen, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl may be optionally substituted with one to four Z⁷;
R⁸ and R⁹ at each occurrence are independently hydrogen, -S(O)₂R¹⁰, -C(O)-R¹⁰, -C(O)O-R¹⁰, -C(O)N(R¹⁰)(R¹¹), C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl may be optionally substituted with one to four Z⁸;
R¹⁰ and R¹¹ at each occurrence are independently hydrogen, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl optionally is substituted with one to four Z^{1b};
each Z¹, Z², Z³, Z⁴, Z⁵, Z⁶, Z⁷, and Z⁸ is independently hydrogen, oxo, halo, -NO₂, -N₃, -CN, thioxo, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -O-R¹²,

   -C(O)-R¹², -C(O)O-R¹², -C(O)-N(R¹³)(R¹⁴), -N(R¹³)(R¹⁴), -N(R¹³)₂(R¹⁴)⁺, -N(R¹²)C(O)-R¹²,

   -N(R¹²)C(O)O-R¹², -N(R¹²)C(O)N(R¹³)(R¹⁴), -N(R¹²)S(O)₂(R¹²), -NR¹²S(O)₂N(R¹³)(R¹⁴),

   -NR¹²S(O)₂O(R¹²), -OC(O)R¹², -OC(O)-N(R¹³)(R¹⁴), -P(O)(OR¹²)₂, -OP(O)(OR¹²)₂, -CH₂P(O)(OR¹²)₂,

   -OCH₂P(O)(OR¹²)₂,

   -C(O)OCH₂P(O)(OR¹²)₂, -P(O)(R¹²)(OR¹²), -OP(O)(R¹²)(OR¹²), -CH₂P(O)(R¹²)(OR¹²),

   -OCH₂P(O)(R¹²)(OR¹²), -C(O)OCH₂P(O)(R¹²)(OR¹²), -P(O)(N(R¹²)₂)₂, -OP(O)(N(R¹²)₂)₂,

   -CH₂P(O)(N(R¹²)₂)₂, -OCH₂P(O)(N(R¹²)₂)₂, -C(O)OCH₂P(O)(N(R¹²)₂)₂, -P(O)(N(R¹²)₂)(OR¹²),

   -OP(O)(N(R¹²)₂)(OR¹²), -CH₂P(O)(N(R¹²)₂)(OR¹²), -OCH₂P(O)(N(R¹²)₂)(OR¹²),

   -C(O)OCH₂P(O)(N(R¹²)₂)(OR¹²), -P(O)(R¹²)(N(R¹²)₂), -OP(O)(R¹²)(N(R¹²)₂), -CH₂P(O)(R¹²)(N(R¹²)₂),

   -OCH₂P(O)(R¹²)(N(R¹²)₂), -C(O)OCH₂P(O)(R¹²)(N(R¹²)₂), -Si(R¹²)₃, -S-R¹², -S(O)R¹², -S(O)(NH)R¹²,

   -S(O)₂R¹² or -S(O)₂N(R¹³)(R¹⁴);
wherein any alkyl, alkenyl, alkynyl, cycloalkyl, haloalkyl, aryl, heteroaryl or heterocyclyl is optionally substituted with one to four Z^{1a} groups;
each Z^{1a} is independently oxo, halo, thioxo, -NO₂, -CN, -N₃, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -O-R¹², -C(O)R¹², -C(O)O-R¹², -C(O)N(R¹³)(R¹⁴), -N(R¹³)(R¹⁴), -N(R¹³)₂(R¹⁴) ⁺, -N(R¹²)-C(O)R¹², -N(R¹²)C(O)O(R¹²), -N(R¹²)C(O)N(R¹³)(R¹⁴), -N(R¹²)S(O)₂(R¹²), -N(R¹²)S(O)₂-N(R¹³)(R¹⁴), -N(R¹²)S(O)₂O(R¹²), -OC(O)R¹², -OC(O)OR¹², -OC(O)-N(R¹³)(R¹⁴), -Si(R¹²)₃, -S-R¹², -S(O)R¹², -S(O)(NH)R¹², -S(O)₂R¹² or -S(O)₂N(R¹³)(R¹⁴); wherein any alkyl, alkenyl, alkynyl, cycloalkyl, aryl, heteroaryl or heterocyclyl is optionally substituted with one to four Z^{1b} groups;
each R¹² is independently hydrogen, C₁₋₉ alkyl, C₂₋₆ alkenyl, C₂₋₆ alkynyl, C₃₋₁₅ cycloalkyl, aryl, heteroaryl or heterocyclyl; wherein any alkyl, alkenyl, alkynyl, cycloalkyl, aryl, heteroaryl or heterocyclyl is optionally substituted with one to four Z^{1b} groups;
R¹³ and R¹⁴ at each occurrence are each independently hydrogen, C₁₋₉ alkyl, C₂₋₆ alkenyl, C₂₋₆ alkynyl, C₃₋₁₅ cycloalkyl, aryl, heteroaryl or heterocyclyl; wherein any alkyl, alkenyl, alkynyl, cycloalkyl, aryl, heteroaryl or heterocyclyl is optionally substituted with one to four Z^{1b} groups, or R¹³ and R¹⁴ together with the nitrogen to which they are attached form a heterocyclyl, wherein said heterocyclyl is optionally substituted with one to four Z^{1b} groups;
each R¹⁵ is independently halo, -CN, -NO₂, -O-R⁷, -N(R⁸)(R⁹), -S(O)-R⁷, -S(O)₂R⁷, -S(O)₂N(R⁷)₂, -C(O)R⁷, -OC(O)-R⁷, -C(O)O-R⁷, -OC(O)O-R⁷, -OC(O)N(R¹⁰)(R¹¹), -C(O)N(R⁷)₂, -N(R⁷)C(O)(R⁷), C₁₋₉ alkyl, C₂₋₆ alkenyl, C₂₋₆ alkynyl, C₁₋₉ alkylthio, C₁₋₆ haloalkyl, C₃₋₁₅ cycloalkyl, aryl, heterocyclyl, or heteroaryl; and
each Z^{1b} is independently oxo, thioxo, hydroxy, halo, -NO₂, -N₃, -CN, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -O(C₁₋₉ alkyl), -O(C₂₋₆ alkenyl),

   -O(C₂₋₆ alkynyl), -O(C₃₋₁₅ cycloalkyl), -O(C₁₋₈ haloalkyl), -O(aryl), -O(heteroaryl), -O(heterocyclyl),

   -NH₂,

   -NH(C₁₋₉ alkyl), -NH(C₂₋₆ alkenyl), -NH(C₂₋₆ alkynyl), -NH(C₃₋₁₅ cycloalkyl), -NH(C₁₋₈ haloalkyl),

   -NH(aryl), -NH(heteroaryl), -NH(heterocyclyl), -N(C₁₋₉ alkyl)₂, -N(C₃₋₁₅ cycloalkyl)₂, -N(C₂₋₆ alkenyl)₂,

   -N(C₂₋₆ alkynyl)₂, -N(C₃₋₁₅ cycloalkyl)₂, -N(C₁₋₈ haloalkyl)₂, -N(aryl)₂, -N(heteroaryl)₂, -N(heterocyclyl)₂,

   -N(C₁₋₉ alkyl)(C₃₋₁₅ cycloalkyl), -N(C₁₋₉ alkyl)(C₂₋₆ alkenyl), -N(C₁₋₉ alkyl)(C₂₋₆ alkynyl),

   -N(C₁₋₉ alkyl)(C₃₋₁₅ cycloalkyl), -N(C₁₋₉ alkyl)(C₁₋₈ haloalkyl), -N(C₁₋₉ alkyl)(aryl),

   -N(C₁₋₉ alkyl)(heteroaryl), -N(C₁₋₉ alkyl)(heterocyclyl), -C(O)(C₁₋₉ alkyl), -C(O)(C₂₋₆ alkenyl),

   -C(O)(C₂₋₆ alkynyl), -C(O)(C₃₋₁₅ cycloalkyl), -C(O)(C₁₋₈ haloalkyl), -C(O)(aryl), -C(O)(heteroaryl),

   -C(O)(heterocyclyl), -C(O)O(C₁₋₉ alkyl), -C(O)O(C₂₋₆ alkenyl), -C(O)O(C₂₋₆ alkynyl),

   -C(O)O(C₃₋₁₅ cycloalkyl), -C(O)O(C₁₋₈ haloalkyl), -C(O)O(aryl), -C(O)O(heteroaryl),

   -C(O)O(heterocyclyl), -C(O)NH₂, -C(O)NH(C₁₋₉ alkyl), -C(O)NH(C₂₋₆ alkenyl), -C(O)NH(C₂₋₆ alkynyl),

   -C(O)NH(C₃₋₁₅ cycloalkyl), -C(O)NH(C₁₋₈ haloalkyl), -C(O)NH(aryl), -C(O)NH(heteroaryl),

   -C(O)NH(heterocyclyl), -C(O)N(C₁₋₉ alkyl)₂, -C(O)N(C₃₋₁₅ cycloalkyl)₂, -C(O)N(C₂₋₆ alkenyl)₂,

   -C(O)N(C₂₋₆ alkynyl)₂, -C(O)N(C₃₋₁₅ cycloalkyl)₂, -C(O)N(C₁₋₈ haloalkyl)₂, -C(O)N(aryl)₂,

   -C(O)N(heteroaryl)₂, -C(O)N(heterocyclyl)₂, -NHC(O)(C₁₋₉ alkyl), -NHC(O)(C₂₋₆ alkenyl),

   -NHC(O)(C₂₋₆ alkynyl), -NHC(O)(C₃₋₁₅ cycloalkyl), -NHC(O)(C₁₋₈ haloalkyl), -NHC(O)(aryl),

   -NHC(O)(heteroaryl), -NHC(O)(heterocyclyl), -NHC(O)O(C₁₋₉ alkyl), -NHC(O)O(C₂₋₆ alkenyl),

   -NHC(O)O(C₂₋₆ alkynyl), -NHC(O)O(C₃₋₁₅ cycloalkyl), -NHC(O)O(C₁₋₈ haloalkyl), -NHC(O)O(aryl),

   -NHC(O)O(heteroaryl), -NHC(O)O(heterocyclyl), -NHC(O)NH(C₁₋₉ alkyl), -NHC(O)NH(C₂₋₆ alkenyl),

   -NHC(O)NH(C₂₋₆ alkynyl), -NHC(O)NH(C₃₋₁₅ cycloalkyl), -NHC(O)NH(C₁₋₈ haloalkyl),

   -NHC(O)NH(aryl), -NHC(O)NH(heteroaryl), -NHC(O)NH(heterocyclyl), -SH, -S(C₁₋₉ alkyl), -S(C₂₋₆ alkenyl), -S(C₂₋₆ alkynyl),

   -S(C₃₋₁₅ cycloalkyl), -S(C₁₋₈ haloalkyl), -S(aryl), -S(heteroaryl), -S(heterocyclyl), -NHS(O)(C₁₋₉ alkyl),

   -N(C₁₋₉ alkyl)(S(O)(C₁₋₉ alkyl), -S(O)N(C₁₋₉ alkyl)₂, -S(O)(C₁₋₉ alkyl), -S(O)(NH)(C₁₋₉ alkyl),

   -S(O)(C₂₋₆ alkenyl), -S(O)(C₂₋₆ alkynyl), -S(O)(C₃₋₁₅ cycloalkyl), -S(O)(C₁₋₈ haloalkyl), -S(O)(aryl),

   -S(O)(heteroaryl), -S(O)(heterocyclyl), -S(O)₂(C₁₋₉ alkyl), -S(O)₂(C₂₋₆ alkenyl), -S(O)₂(C₂₋₆ alkynyl),

   -S(O)₂(C₃₋₁₅ cycloalkyl), -S(O)₂(C₁₋₈ haloalkyl), -S(O)₂(aryl), -S(O)₂(heteroaryl), -S(O)₂(heterocyclyl),

   -S(O)₂NH(C₁₋₉ alkyl), or -S(O)₂N(C₁₋₉ alkyl)₂;
wherein any alkyl, cycloalkyl, aryl, heteroaryl, or heterocyclyl is optionally substituted with one to four halo, C₁₋₉ alkyl, C₁₋₈ haloalkyl, -OH, -NH₂, -NH(C₁₋₉ alkyl), -NH(C₃₋₁₅ cycloalkyl), -NH(C₁₋₈ haloalkyl), -NH(aryl), -NH(heteroaryl), -NH(heterocyclyl), -N(C₁₋₉ alkyl)₂, -N(C₃₋₁₅ cycloalkyl)₂, -NHC(O)(C₃₋₁₅ cycloalkyl), -NHC(O)(C₁₋₈ haloalkyl), -NHC(O)(aryl), -NHC(O)(heteroaryl), -NHC(O)(heterocyclyl), -NHC(O)O(C₁₋₉ alkyl), -NHC(O)O(C₂₋₆ alkynyl), -NHC(O)O(C₃₋₁₅ cycloalkyl), -NHC(O)O(C₁₋₈ haloalkyl), -NHC(O)O(aryl), -NHC(O)O(heteroaryl), -NHC(O)O(heterocyclyl), -NHC(O)NH(C₁₋₉ alkyl), -S(O)(NH)(C₁₋₉ alkyl), S(O)₂(C₁₋₉ alkyl), -S(O)₂(C₃₋₁₅ cycloalkyl), -S(O)₂(C₁₋₈ haloalkyl), -S(O)z(aryl), -S(O)₂(heteroaryl), -S(O)₂(heterocyclyl), -S(O)₂NH(C₁₋₉ alkyl), -S(O)₂N(C₁₋₉ alkyl)₂, -O(C₃₋₁₅ cycloalkyl), -O(C₁₋₈ haloalkyl), -O(aryl), -O(heteroaryl), -O(heterocyclyl), or
   -O(C₁₋₉ alkyl); and
m is 0, 1, or 2.

In one example, the COT inhibitor is a compound having structure of Formula I, R⁴ is heterocyclyl or heteroaryl, wherein each heterocyclyl or heteroaryl is optionally substituted with one to four Z⁴. In one example, the COT inhibitor is a compound having structure of Formula I, R⁴ is heterocyclyl, wherein each heterocyclyl is optionally substituted with one to four Z⁴. In one example, the COT inhibitor is a compound having structure of Formula I, R⁴ is heteroaryl, wherein each heteroaryl is optionally substituted with one to four Z⁴. In one example, the COT inhibitor is a compound having structure of Formula I, R⁴ is aryl, wherein said aryl is optionally substituted with one to four Z⁴.

The compound that inhibits COT may be a compound of Formula II: 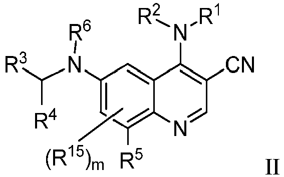 or a pharmaceutically acceptable salt, stereoisomer, mixture of stereoisomers, or deuterated analog thereof, wherein:
R¹ is hydrogen, -O-R⁷, -N(R⁸)(R⁹), -C(O)-R⁷, -S(O)₂-R⁷, -C₁₋₉ alkyl, C₂₋₆ alkenyl, C₂₋₆ alkynyl, C₃₋₁₅ cycloalkyl, heterocyclyl, aryl, or heteroaryl; wherein each C₁₋₉ alkyl, C₂₋₆ alkenyl, C₂₋₆ alkynyl, C₃₋₁₅ cycloalkyl, heterocyclyl, aryl, and heteroaryl may be optionally substituted with one to four Z¹;
R² is hydrogen, -C(O)-R⁷, -C(O)O-R⁷, -C(O)N(R⁷)₂, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl may be optionally substituted with one to four Z²;
or R¹ and R² together with the nitrogen to which they are attached to form a heterocyclyl or heteroaryl, wherein each heterocyclyl or heteroaryl is optionally substituted with one to four Z²;
R³ is heterocyclyl or heteroaryl, wherein each heterocyclyl or heteroaryl is optionally substituted with one to four Z³;
R⁴ is aryl, heterocyclyl or heteroaryl, wherein each aryl, heterocyclyl, or heteroaryl is optionally substituted with one to four Z⁴;
R⁵ is hydrogen, halo, -CN, -NO₂, -O-R⁷, -N(R⁸)(R⁹), -S(O)-R⁷, -S(O)₂R⁷, -S(O)₂N(R⁷)₂, -C(O)R⁷, -OC(O)-R⁷, -C(O)O-R⁷, -OC(O)O-R⁷, -OC(O)N(R¹⁰)(R¹¹), -C(O)N(R⁷)₂, -N(R⁷)C(O)(R⁷), C₁₋₉ alkyl, C₂₋₆ alkenyl, C₂₋₆ alkynyl, C₁₋₉ alkylthio, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₉ alkylthio, C₁₋₆ haloalkyl, C₃₋₁₅ cycloalkyl, aryl, heterocyclyl, and heteroaryl may be optionally substituted with one to four Z⁵;
R⁶ is -C(O)O-R¹⁶-OP(O)(OR¹²)₂ -C(O)-R¹⁶-OP(O)(OR¹²)₂, -R¹⁶-OP(O)(OR¹²)₂, -C(O)O-R¹⁶-OR¹⁷; -C(O)O-R¹⁶-OH; -C(O)O-R¹⁶-OC(O)R¹⁷; -C(O)-C(O)OR¹², or -C(O)O- R¹⁶-OC(O)R¹⁷NH₂;
each R⁷ is independently hydrogen, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl may be optionally substituted with one to four Z⁷;
R⁸ and R⁹ at each occurrence are independently hydrogen, -S(O)₂R¹⁰, -C(O)-R¹⁰, -C(O)O-R¹⁰, -C(O)N(R¹⁰)(R¹¹), C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl may be optionally substituted with one to four Z⁸;
R¹⁰ and R¹¹ at each occurrence are independently hydrogen, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl optionally is substituted with one to four Z^{1b};
each Z¹, Z², Z³, Z⁴, Z⁵, Z⁶, Z⁷, and Z⁸ is independently hydrogen, oxo, halo, -NO₂, -N₃, -CN, thioxo, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -OR¹²,

   -C(O)-R¹², -C(O)O-R¹², -C(O)-N(R¹³)(R¹⁴), -N(R¹³)(R¹⁴), -N(R¹³)₂(R¹⁴)⁺, -N(R¹²)C(O)-R¹², -N(R¹²)C(O)O-R¹², -N(R¹²)C(O)N(R¹³)(R¹⁴), -N(R¹²)S(O)₂(R¹²), -NR¹²S(O)₂N(R¹³)(R¹⁴), -NR¹²S(O)₂O(R¹²), -OC(O)R¹²,

   -OC(O)-N(R¹³)(R¹⁴), -P(O)(OR¹²)₂, -OP(O)(OR¹²)₂, -CH₂P(O)(OR¹²)₂, -OCH₂P(O)(OR¹²)₂,

   -C(O)OCH₂P(O)(OR¹²)₂, -P(O)(R¹²)(OR¹²), -OP(O)(R¹²)(OR¹²), -CH₂P(O)(R¹²)(OR¹²),

   -OCH₂P(O)(R¹²)(OR¹²), -C(O)OCH₂P(O)(R¹²)(OR¹²), -P(O)(N(R¹²)₂)₂, -OP(O)(N(R¹²)₂)₂,

   -CH₂P(O)(N(R¹²)₂)₂, -OCH₂P(O)(N(R¹²)₂)₂, -C(O)OCH₂P(O)(N(R¹²)₂)₂, -P(O)(N(R¹²)₂)(OR¹²),

   -OP(O)(N(R¹²)₂)(OR¹²),

   -CH₂P(O)(N(R¹²)₂)(OR¹²), -OCH₂P(O)(N(R¹²)₂)(OR¹²), -C(O)OCH₂P(O)(N(R¹²)₂)(OR¹²),

   -P(O)(R¹²)(N(R¹²)₂), -OP(O)(R¹²)(N(R¹²)₂), -CH₂P(O)(R¹²)(N(R¹²)₂), -OCH₂P(O)(R¹²)(N(R¹²)₂),

   -C(O)OCH₂P(O)(R¹²)(N(R¹²)₂),

   -Si(R¹²)₃, -S-R¹², -S(O)R¹², -S(O)(NH)R¹², -S(O)₂R¹² or -S(O)₂N(R¹³)(R¹⁴); wherein any alkyl, alkenyl, alkynyl, cycloalkyl, haloalkyl, aryl, heteroaryl or heterocyclyl is optionally substituted with one to four Z^{1a} groups;
each Z^{1a} is independently oxo, halo, thioxo, -NO₂, -CN, -N₃, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -O-R¹², -C(O)R¹², -C(O)O-R¹², -C(O)N(R¹³)(R¹⁴), -N(R¹³)(R¹⁴), -N(R¹³)₂(R¹⁴) ⁺, -N(R¹²)-C(O)R¹², -N(R¹²)C(O)O(R¹²), -N(R¹²)C(O)N(R¹³)(R¹⁴), -N(R¹²)S(O)₂(R¹²), -N(R¹²)S(O)₂-N(R¹³)(R¹⁴), -N(R¹²)S(O)₂O(R¹²), -OC(O)R¹², -OC(O)OR¹², -OC(O)-N(R¹³)(R¹⁴), -Si(R¹²)₃, -S-R¹², -S(O)R¹², -S(O)(NH)R¹², -S(O)₂R¹² or -S(O)₂N(R¹³)(R¹⁴); wherein any alkyl, alkenyl, alkynyl, cycloalkyl, aryl, heteroaryl or heterocyclyl is optionally substituted with one to four Z^{1b} groups;
each R¹² is independently hydrogen, C₁₋₉ alkyl, C₂₋₆ alkenyl, C₂₋₆ alkynyl, C₃₋₁₅ cycloalkyl, aryl, heteroaryl or heterocyclyl; wherein any alkyl, alkenyl, alkynyl, cycloalkyl, aryl, heteroaryl or heterocyclyl is optionally substituted with one to four Z^{1b} groups;
R¹³ and R¹⁴ at each occurrence are each independently hydrogen, C₁₋₉ alkyl, C₂₋₆ alkenyl, C₂₋₆ alkynyl, C₃₋₁₅ cycloalkyl, aryl, heteroaryl or heterocyclyl; wherein any alkyl, alkenyl, alkynyl, cycloalkyl, aryl, heteroaryl or heterocyclyl is optionally substituted with one to four Z^{1b} groups, or R¹³ and R¹⁴ together with the nitrogen to which they are attached form a heterocyclyl, wherein said heterocyclyl is optionally substituted with one to four Z^{1b} groups;
each R¹⁵ is independently halo, -CN, -NO₂, -O-R⁷, -N(R⁸)(R⁹), -S(O)-R⁷, -S(O)₂R⁷, -S(O)₂N(R⁷)₂, -C(O)R⁷, -OC(O)-R⁷, -C(O)O-R⁷, -OC(O)O-R⁷, -OC(O)N(R¹⁰)(R¹¹), -C(O)N(R⁷)₂, -N(R⁷)C(O)(R⁷), C₁₋₉ alkyl, C₂₋₆ alkenyl, C₂₋₆ alkynyl, C₁₋₉ alkylthio, C₁₋₆ haloalkyl, C₃₋₁₅ cycloalkyl, aryl, heterocyclyl, or heteroaryl;
R¹⁶ is -C₁₋₃ alkyl or cyclopropyl optionally substituted with one to four C₁₋₃ alkyl or cyclopropyl;
R¹⁷ is C₁₋₉ alkyl, cycloalkyl, or heterocyclyl optionally substituted with one to three R¹⁶; and
each Z¹, Z², Z⁴, Z⁵, Z⁷, and Z⁸ is independently hydrogen, oxo, halo, -NO₂, -N₃, -CN, thioxo, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -O-R¹²,

   -C(O)-R¹²,

   -C(O)O-R¹², -C(O)-N(R¹³)(R¹⁴), -N(R¹³)(R¹⁴), -N(R¹³)₂(R¹⁴)⁺, -N(R¹²)C(O)-R¹², -N(R¹²)C(O)O-R¹²,

   -N(R¹²)C(O)N(R¹³)(R¹⁴), -N(R¹²)S(O)₂(R¹²), -NR¹²S(O)₂N(R¹³)(R¹⁴), -NR¹²S(O)₂O(R¹²), -OC(O)R¹²,

   -OC(O)-N(R¹³)(R¹⁴), -P(O)(OR¹²)₂, -OP(O)(OR¹²)₂, -CH₂P(O)(OR¹²)₂, -OCH₂P(O)(OR¹²)₂,

   -C(O)OCH₂P(O)(OR¹²)₂, -P(O)(R¹²)(OR¹²), -OP(O)(R¹²)(OR¹²), -CH₂P(O)(R¹²)(OR¹²),

   -OCH₂P(O)(R¹²)(OR¹²), -C(O)OCH₂P(O)(R¹²)(OR¹²), -P(O)(N(R¹²)₂)₂, -OP(O)(N(R¹²)₂)₂,

   -CH₂P(O)(N(R¹²)₂)₂, -OCH₂P(O)(N(R¹²)₂)₂, -C(O)OCH₂P(O)(N(R¹²)₂)₂, -P(O)(N(R¹²)₂)(OR¹²),

   -OP(O)(N(R¹²)₂)(OR¹²),

   -CH₂P(O)(N(R¹²)₂)(OR¹²), -OCH₂P(O)(N(R¹²)₂)(OR¹²), -C(O)OCH₂P(O)(N(R¹²)₂)(OR¹²),

   -P(O)(R¹²)(N(R¹²)₂), -OP(O)(R¹²)(N(R¹²)₂), -CH₂P(O)(R¹²)(N(R¹²)₂), -OCH₂P(O)(R¹²)(N(R¹²)₂),

   -C(O)OCH₂P(O)(R¹²)(N(R¹²)₂),

   -Si(R¹²)₃, -S-R¹², -S(O)R¹², -S(O)(NH)R¹², -S(O)₂R¹² or -S(O)₂N(R¹³)(R¹⁴);
wherein any alkyl, alkenyl, alkynyl, cycloalkyl, haloalkyl, aryl, heteroaryl or heterocyclyl is optionally substituted with one to four Z^{1a} groups;
Z⁹ is hydrogen, halo, -CN, or -O-R¹²;
each Z^{1a} is independently oxo, halo, thioxo, -NO₂, -CN, -N₃, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -O-R¹², -C(O)R¹², -C(O)O-R¹²,

   -C(O)N(R¹³)(R¹⁴), -N(R¹³)(R¹⁴), -N(R¹³)₂(R¹⁴) ⁺, -N(R¹²)-C(O)R¹², -N(R¹²)C(O)O(R¹²),

   -N(R¹²)C(O)N(R¹³)(R¹⁴), -N(R¹²)S(O)₂(R¹²), -N(R¹²)S(O)₂-N(R¹³)(R¹⁴), -N(R¹²)S(O)₂O(R¹²), -OC(O)R¹²,

   -OC(O)OR¹², -OC(O)-N(R¹³)(R¹⁴), -Si(R¹²)₃, -S-R¹², -S(O)R¹², -S(O)(NH)R¹², -S(O)₂R¹²

   or

   -S(O)₂N(R¹³)(R¹⁴);
wherein any alkyl, alkenyl, alkynyl, cycloalkyl, aryl, heteroaryl or heterocyclyl is optionally substituted with one to four Z^{1b} groups;
each Z^{1b} is independently oxo, thioxo, hydroxy, halo, -NO₂, -N₃, -CN, C₁₋₉ alkyl, C₂₋₆ alkenyl, C₂₋₆ alkynyl, C₃₋₁₅ cycloalkyl, C₁₋₈ haloalkyl, aryl, heteroaryl, heterocyclyl, -O(C₁₋₉ alkyl), -O(C₂₋₆ alkenyl),

   -O(C₂₋₆ alkynyl), -O(C₃₋₁₅ cycloalkyl), -O(C₁₋₈ haloalkyl), -O(aryl), -O(heteroaryl), - O(heterocyclyl),

   -NH₂,

   -NH(C₁₋₉ alkyl), -NH(C₂₋₆ alkenyl), -NH(C₂₋₆ alkynyl), -NH(C₃₋₁₅ cycloalkyl), -NH(C₁₋₈ haloalkyl),

   -NH(aryl), -NH(heteroaryl), -NH(heterocyclyl), -N(C₁₋₉ alkyl)₂, -N(C₂₋₆ alkenyl)₂, -N(C₂₋₆ alkynyl)₂,

   -N(C₃₋₁₅ cycloalkyl)₂, -N(C₁₋₈ haloalkyl)₂, -N(aryl)₂, -N(heteroaryl)₂, -N(heterocyclyl)₂,

   -N(C₁₋₉ alkyl)(C₂₋₆ alkenyl), -N(C₁₋₉ alkyl)(C₂₋₆ alkynyl), -N(C₁₋₉ alkyl)(C₃₋₁₅ cycloalkyl),

   -N(C₁₋₉ alkyl)(C₁₋₈ haloalkyl), -N(C₁₋₉ alkyl)(aryl), -N(C₁₋₉ alkyl)(heteroaryl), -N(C₁₋₉ alkyl)(heterocyclyl),

   -C(O)(C₁₋₉ alkyl), -C(O)(C₂₋₆ alkenyl), -C(O)(C₂₋₆ alkynyl), -C(O)(C₃₋₁₅ cycloalkyl), -C(O)(C₁₋₈ haloalkyl),

   -C(O)(aryl), -C(O)(heteroaryl), -C(O)(heterocyclyl), -C(O)O(C₁₋₉ alkyl), -C(O)O(C₂₋₆ alkenyl),

   -C(O)O(C₂₋₆ alkynyl), -C(O)O(C₃₋₁₅ cycloalkyl), -C(O)O(C₁₋₈ haloalkyl), -C(O)O(aryl),

   -C(O)O(heteroaryl), -C(O)O(heterocyclyl), -C(O)NH₂, -C(O)NH(C₁₋₉ alkyl), -C(O)NH(C₂₋₆ alkenyl),

   -C(O)NH(C₂₋₆ alkynyl),

   -C(O)NH(C₃₋₁₅ cycloalkyl), -C(O)NH(C₁₋₈ haloalkyl), -C(O)NH(aryl), -C(O)NH(heteroaryl),

   -C(O)NH(heterocyclyl), -C(O)N(C₁₋₉ alkyl)₂, -C(O)N(C₂₋₆ alkenyl)₂, -C(O)N(C₂₋₆ alkynyl)₂,

   -C(O)N(C₃₋₁₅ cycloalkyl)₂, -C(O)N(C₁₋₈ haloalkyl)₂, -C(O)N(aryl)₂, -C(O)N(heteroaryl)₂,

   -C(O)N(heterocyclyl)₂, -NHC(O)(C₁₋₉ alkyl), -NHC(O)(C₂₋₆ alkenyl), -NHC(O)(C₂₋₆ alkynyl),

   -NHC(O)(C₃₋₁₅ cycloalkyl), -NHC(O)(C₁₋₈ haloalkyl), -NHC(O)(aryl), -NHC(O)(heteroaryl),

   -NHC(O)(heterocyclyl), -NHC(O)O(C₁₋₉ alkyl), -NHC(O)O(C₂₋₆ alkenyl), -NHC(O)O(C₂₋₆ alkynyl),

   -NHC(O)O(C₃₋₁₅ cycloalkyl), -NHC(O)O(C₁₋₈ haloalkyl), -NHC(O)O(aryl), -NHC(O)O(heteroaryl),

   -NHC(O)O(heterocyclyl), -NHC(O)NH(C₁₋₉ alkyl), -NHC(O)NH(C₂₋₆ alkenyl), -NHC(O)NH(C₂₋₆ alkynyl),

   -NHC(O)NH(C₃₋₁₅ cycloalkyl), -NHC(O)NH(C₁₋₈ haloalkyl), -NHC(O)NH(aryl),

   -NHC(O)NH (heteroaryl),

   -NHC(O)NH(heterocyclyl), -SH, -S(C₁₋₉ alkyl), -S(C₂₋₆ alkenyl), -S(C₂₋₆ alkynyl), -S(C₃₋₁₅ cycloalkyl),

   -S(C₁₋₈ haloalkyl), -S(aryl), -S(heteroaryl), -S(heterocyclyl), -NHS(O)(C₁₋₉ alkyl),

   -N(C₁₋₉ alkyl)S(O)(C₁₋₉ alkyl), -S(O)N(C₁₋₉ alkyl)₂, -S(O)(C₁₋₉ alkyl), -S(O)(NH)(C₁₋₉ alkyl),

   -S(O)(C₂₋₆ alkenyl), -S(O)(C₂₋₆ alkynyl), -S(O)(C₃₋₁₅ cycloalkyl), -S(O)(C₁₋₈ haloalkyl), -S(O)(aryl),

   -S(O)(heteroaryl), -S(O)(heterocyclyl), -S(O)₂(C₁₋₉ alkyl), -S(O)₂(C₂₋₆ alkenyl), -S(O)₂(C₂₋₆ alkynyl),

   -S(O)₂(C₃₋₁₅ cycloalkyl), -S(O)₂(C₁₋₈ haloalkyl), -S(O)₂(aryl), -S(O)₂(heteroaryl), -S(O)₂(heterocyclyl),

   -S(O)₂NH(C₁₋₉ alkyl), or -S(O)₂N(C₁₋₉ alkyl)₂;
wherein any alkyl, cycloalkyl, aryl, heteroaryl, or heterocyclyl is optionally substituted with one to four halo, C₁₋₉ alkyl, C₁₋₈ haloalkyl, -OH, -NH₂, -NH(C₁₋₉ alkyl), -NH(C₃₋₁₅ cycloalkyl), -NH(C₁₋₈ haloalkyl), -NH(aryl), -NH(heteroaryl), -NH(heterocyclyl), -N(C₁₋₉ alkyl)₂, -N(C₃₋₁₅ cycloalkyl)₂, -NHC(O)(C₃₋₁₅ cycloalkyl), -NHC(O)(C₁₋₈ haloalkyl), -NHC(O)(aryl), -NHC(O)(heteroaryl), -NHC(O)(heterocyclyl), -NHC(O)O(C₁₋₉ alkyl), -NHC(O)O(C₂₋₆ alkynyl), -NHC(O)O(C₃₋₁₅ cycloalkyl), -NHC(O)O(C₁₋₈ haloalkyl), -NHC(O)O(aryl), -NHC(O)O(heteroaryl), -NHC(O)O(heterocyclyl), -NHC(O)NH(C₁₋₉ alkyl), -S(O)(NH)(C₁₋₉ alkyl), S(O)₂(C₁₋₉ alkyl), -S(O)₂(C₃₋₁₅ cycloalkyl), -S(O)₂(C₁₋₈ haloalkyl), -S(O)₂(aryl), -S(O)₂(heteroaryl), -S(O)₂(heterocyclyl), -S(O)₂NH(C₁₋₉ alkyl), -S(O)₂N(C₁₋₉ alkyl)₂, -O(C₃₋₁₅ cycloalkyl), -O(C₁₋₈ haloalkyl), -O(aryl), -O(heteroaryl), -O(heterocyclyl), or

   -O(C₁₋₉ alkyl);
m is 0, 1, or 2.

The compound that inhibits COT may be a compound of Formula III: 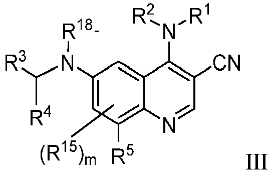 wherein
R¹, R², R³, R⁴, R⁵, R¹⁵, and m are as described herein, and
R¹⁸ is hydrogen, -C(O)-R⁷, -C(O)O-R⁷, -C(O)N(R⁷)₂, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl may be optionally substituted with one to four Z⁶;
each R⁷ is independently hydrogen, C₁₋₉ alkyl, C₂₋₆ alkenyl, C₂₋₆ alkynyl, C₁₋₆ haloalkyl, C₃₋₁₅ cycloalkyl, aryl, heterocyclyl, or heteroaryl; wherein each C₁₋₉ alkyl, C₂₋₆ alkenyl, C₂₋₆ alkynyl, C₁₋₆ haloalkyl, C₃₋₁₅ cycloalkyl, aryl, heterocyclyl, and heteroaryl may be optionally substituted with one to four Z⁷;
where each Z⁶ and Z⁷ are independently as defined herein;
or a pharmaceutically acceptable salt, tautomer, stereoisomer, mixture of stereoisomers, prodrug, or deuterated analog thereof.

The compound that inhibits COT may be a compound of Formula IV: 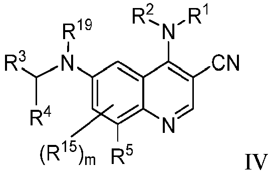 wherein
R¹, R², R³, R⁴, R⁵, R¹⁵, and m are as described herein, and
R¹⁹ is -C(O)O-R¹⁶-OP(O)(OR¹²)₂ -C(O)-R¹⁶-OP(O)(OR¹²)₂, -R¹⁶-OP(O)(OR¹²)₂, -C(O)O-R¹⁶-OR¹⁷; -C(O)O-R¹⁶-OH; -C(O)O-R¹⁶-OC(O)R¹⁷; -C(O)-C(O)OR¹², or -C(O)O- R¹⁶-OC(O)R¹⁷NH₂;
where each R¹², R¹⁶, and R¹⁷ are independently as defined herein;
or a pharmaceutically acceptable salt, tautomer, stereoisomer, mixture of stereoisomers, prodrug, or deuterated analog thereof.

In certain embodiments of the present invention, the compound that inhibits COT is: 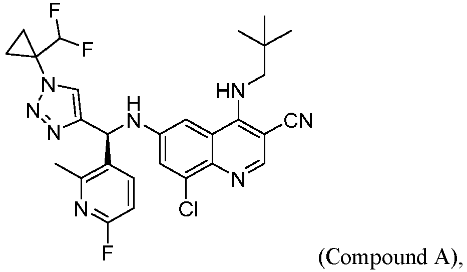 or a pharmaceutically acceptable salt thereof.

Also included are optical isomers, racemates, or other mixtures thereof of the compounds described herein or pharmaceutically acceptable salts or a mixture thereof. In those situations, the single enantiomer or diastereomer, *i.e.,* optically active form, can be obtained by asymmetric synthesis or by resolution. Resolution can be accomplished, for example, by conventional methods such as crystallization in the presence of a resolving agent, or chromatography, using for example, a chiral high pressure liquid chromatography (HPLC) column.

Compositions provided herein can include a compound described herein or pharmaceutically acceptable salts, isomer, or a mixture thereof may include racemic mixtures, or mixtures containing an enantiomeric excess of one enantiomer or single diastereomers or diastereomeric mixtures. All such isomeric forms of these compounds are expressly included herein the same as if each and every isomeric form were specifically and individually listed.

A composition comprising a mixture of enantiomers (or diastereomers) of a compound described herein or a pharmaceutically acceptable salt thereof, is also provided herein. In some embodiments, the composition comprises a single enantiomer of the compound and is substantially free of the other enantiomer. In certain embodiments, the compound of Formula I (or another Formula as described herein) contains one or more additional stereogenic atom(s) (e.g., at R¹ and/or R³). In such instances, the composition may contain a mixture of diastereomers. In some embodiments, the composition comprises a single enantiomer of the compound and is substantially free (i.e., having less than or 40%, 30%, 25%, 20%, 15%, 10%, 5%, 1%, 0.05%, or 0.01%) of one or more diastereomers.

Accordingly, in certain embodiments, provided is a composition comprising a mixture of Formula IA-1, or a pharmaceutically acceptable salt thereof, and Formula IB-1, or a pharmaceutically acceptable salt thereof. 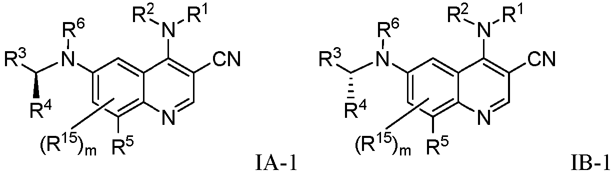 wherein m, R¹, R², R³, R⁴, R⁵, R⁶ and R¹⁵ are as defined herein. In certain embodiments, R⁶ is R¹⁸. In certain embodiments, R⁶ is R¹⁹.

In one embodiment, the mixture is a racemic mixture. In other embodiments, the composition comprises a mixture of Formula IA-1, or a pharmaceutically acceptable salt thereof, and Formula IB-1, or a pharmaceutically acceptable salt thereof, wherein Formula IA-1 is present in excess of over Formula IB-1, or a pharmaceutically acceptable salt thereof. In certain embodiments, provided is a composition substantially free of Formula IB-1, having less than or 40%, 30%, 25%, 20%, 15%, 10%, 5%, 1%, 0.05%, or 0.01% of compounds of Formula IB-1.

In other embodiments, the mixture comprises compounds of Formula IA-1 and IB-1 in a molar ratio of at least or 3:1, at least or 4:1, at least or 5:1, at least or 6:1, at least or 7:1, at least or 8:1, at least or 9:1, at least or 10:1, at least or 11:1, at least or 12:1, at least or 20:1, at least or 30:1, at least or 40:1, at least or 80:1, at least or 160:1, or at least or 320:1, respectively.

In certain embodiments, provided are also chelates, non-covalent complexes, and mixtures thereof, of the compounds described herein or a pharmaceutically acceptable salt, tautomer, stereoisomer, mixture of stereoisomers, or deuterated analog thereof. A "chelate" is formed by the coordination of a compound to a metal ion at two (or more) points. A "non-covalent complex" is formed by the interaction of a compound and another molecule wherein a covalent bond is not formed between the compound and the molecule. For example, complexation can occur through van der Waals interactions, hydrogen bonding, and electrostatic interactions (also called ionic bonding).

In certain embodiments of the methods described herein, the compound that inhibits COT is administered in combination with one or more additional therapeutic agents. In certain embodiments, the one or more additional therapeutic agents is administered prior to, subsequently to, or concurrently with the compound that inhibits COT. In certain embodiments, the compound that inhibits COT is administered in a in a single composition, formulation, or unit dosage form which further comprises one or more additional therapeutic agents.

In certain embodiments of the methods described herein, the compound that inhibits COT is administered in combination with an effective amount of an ACC inhibitor. Exemplary ACC inhibitors for use in the methods disclosed herein can be found in U.S. 9,453,026 and WO 2021/030142.

In certain embodiments, the ACC inhibitor is a compound of Formula IA: 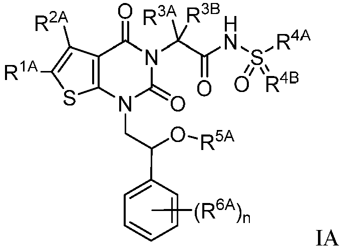 or a pharmaceutically acceptable salt thereof, wherein:
R^{1A} is cyano, halogen, -R, -OR, -SR, -N(R)₂, -N(R)C(O)R, -C(O)N(R)₂, -N(R)C(O)N(R)₂, -N(R)C(O)OR, -OC(O)N(R)₂, -N(R)SO₂R, -SO₂N(R)₂, -C(O)R, -C(O)OR, -OC(O)R, -S(O)R, -SO₂R, or a cyclic group selected from a 4-8 membered monocyclic heterocycle having 1-2 heteroatoms independently selected from nitrogen, oxygen, or sulfur, a 5-6 membered monocyclic heteroaryl having 1-4 heteroatoms independently selected from nitrogen, oxygen, or sulfur, or a 8-10 membered bicyclic heteroaryl having 1-5 heteroatoms independently selected from nitrogen, oxygen, or sulfur, wherein each cyclic group is independently optionally substituted with 1-4 substituents independently selected from halogen, cyano, C₁₋₃ alkyl, and C₁₋₃ alkoxy;
R^{2A} is hydrogen or C₁₋₄ alkyl, optionally substituted with one or more halogen, -OR, -SR, -N(R)₂, -N(R)C(O)R, -C(O)N(R)₂, -N(R)C(O)N(R)₂, -N(R)C(O)OR, -OC(O)N(R)₂, -N(R)SO₂R, -SO₂N(R)₂, -C(O)R, -C(O)OR, -OC(O)R, -S(O)R, or -SO₂R;
each R is independently hydrogen or a group selected from C₁₋₆ alkyl, 3-8 membered monocyclic cycloalkyl, phenyl, 8-10 membered bicyclic aryl; 4-8 membered monocyclic heteroaryl having 1-2 heteroatoms independently selected from nitrogen, oxygen, or sulfur, 5-6 membered monocyclic heteroaryl having 1-4 heteroatoms independently selected from nitrogen, oxygen, or sulfur, and 8-10 membered bicyclic heteroaryl having 1-5 heteroatoms independently selected from nitrogen, oxygen, or sulfur, wherein each group is optionally substituted with 1-4 substituents independently selected from halogen, cyano, C₁₋₃ alkyl, and C₁₋₃ alkoxy;
R^{3A} and R^{3B} are each independently hydrogen or a C₁₋₃ alkyl optionally substituted with 1 to 3 substituents independently selected from halogen, cyano, C₁₋₃ alkyl, or C₁₋₃ alkoxy; or
R^{3A} and R^{3B} together with the carbon to which they are attached form cyclopropylenyl, cyclobutylenyl, oxetanyl, or tetrahydrofuranyl, each optionally substituted with 1 to 3 substituents independently selected from halogen, cyano, C₁₋₃ alkyl, or C₁₋₃ alkoxy;
R^{4A} is C₁₋₆ alkyl, C₁₋₆ alkoxy, C₃₋₆ cycloalkyl, C₃₋₆ cycloalkoxy, phenyl or phenoxy, each optionally substituted with 1 to 3 substituents independently selected from halogen, cyano, C₁₋₃ alkyl, C₁₋₆ alkoxy, C₃₋₆ cycloalkyl, or C₃₋₆ cycloalkoxy, wherein each C₁₋₃ alkyl, C₁₋₆ alkoxy, C₃₋₆ cycloalkyl, or C₃₋₆ cycloalkoxy is optionally substituted with one C₁₋₃ alkoxy or 1 to 3 halogens; or
R^{4A} is -OR⁴¹, wherein R⁴¹ is a 5-6 membered heteroaryl having 1-2 heteroatoms independently selected from nitrogen, oxygen, or sulfur, optionally substituted with 1 to 3 substituents independently selected from halogen, cyano, or C₁₋₃ alkyl; or
R^{4A} is -N(R⁴²)₂, wherein each R⁴² is independently selected from hydrogen, C₁₋₆ alkyl, or C₃₋₆ cycloalkyl; or two R⁴² together with the nitrogen to which they are attached to form a 4-6 membered heterocycle, optionally substituted with one C₁₋₃ alkoxy or 1 to 3 halogens;
R^{4B} is oxo or =NR⁴³, wherein R⁴³ is C₁₋₆ alkyl, C₃₋₆ cycloalkyl, C₄₋₆ heterocyclyl, C₃₋₆ cycloalkoxy, phenyl, or 5-6 membered heteroaryl having 1-2 heteroatoms independently selected from nitrogen, oxygen, or sulfur, wherein each R⁴³ is optionally substituted with 1 to 3 substituents independently selected from halogen, cyano, or C₁₋₃ alkyl;
R^{5A} is an 6-12 membered fused, bridged, or spiro heterocycle having 1 or 2 heteroatoms independently selected from oxygen, sulfur, or nitrogen, wherein the fused, bridged, or spiro heterocycle is optionally substituted with 1 to 4 substituents independently selected from halogen, hydroxyl, oxo, amino, cyano, -OR⁵¹, -SR⁵¹, -N(R⁵¹)_{2,} -N(R⁵¹)C(O)R⁵¹, -C(O)N(R⁵¹)₂, -N(R⁵¹)C(O)N(R⁵¹)₂, -N(R⁵¹)C(O)OR⁵¹,
   -OC(O)N(R⁵¹)₂, -N(R⁵¹)SO₂R⁵¹, -SO₂N(R⁵¹)₂, -C(O)R⁵¹, -C(O)OR⁵¹, -OC(O)R⁵¹, -S(O)R⁵¹, or -SO₂R, C₁₋₃ alkyl, C₁₋₃ alkoxy, wherein each R⁵¹ is independently hydrogen or C₁₋₃ alkyl;
R^{6A} is hydrogen, halogen C₁₋₃ alkyl, C₁₋₆ alkoxy, C₃₋₆ cycloalkyl, or C₃₋₆ cycloalkoxy, wherein the C₁₋₃ alkyl, C₁₋₆ alkoxy, C₃₋₆ cycloalkyl, or C₃₋₆ cycloalkoxy is optionally substituted with one -O-CH₃ or 1 to 3 halogens; and
n is 1, 2, or 3.

Exemplary ACC inhibitors include 4-(4-[(1-isopropyl-7-oxo-1,4,6,7-tetrahydro-1'H-spiro[indazole-5,4'-piperidin]-1'-yl)carbonyl]-6-methoxypyridin-2-yl)benzoic acid or firsocostat (GS-0976, see e.g., U.S. 9,453,026), or a pharmaceutically acceptable salt thereof.

In certain embodiments of the present invention, the ACC inhibitor is: 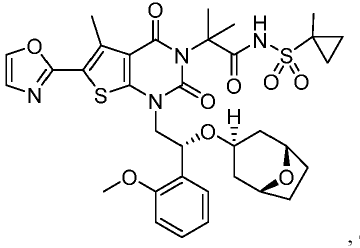 or a pharmaceutically acceptable salt thereof.

In certain embodiments of the present invention, the ACC inhibitor is: 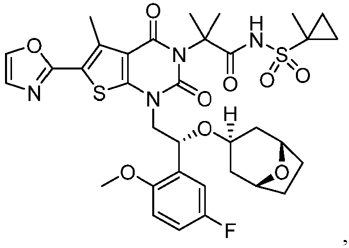 or a pharmaceutically acceptable salt thereof.

In certain embodiments of the present invention, the ACC inhibitor is: 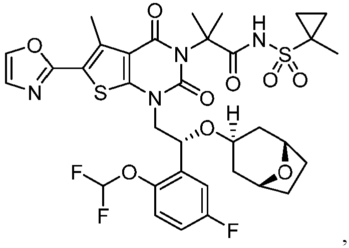 or a pharmaceutically acceptable salt thereof.

In certain embodiments of the present invention, the ACC inhibitor is: 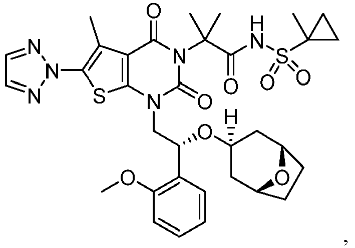 or a pharmaceutically acceptable salt thereof.

In certain embodiments of the present invention, the ACC inhibitor is: 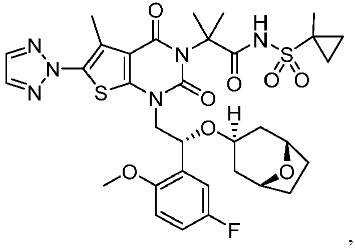 or a pharmaceutically acceptable salt thereof.

In certain embodiments of the present invention, the ACC inhibitor is: 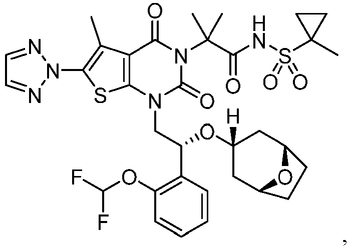 or a pharmaceutically acceptable salt thereof.

In certain embodiments of the present invention, the ACC inhibitor is: 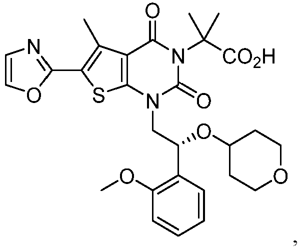 or a pharmaceutically acceptable salt thereof.

In certain embodiments of the present invention, the ACC inhibitor is: 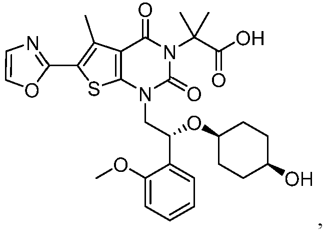 or a pharmaceutically acceptable salt thereof.

Described herein is a method of treating, stabilizing, or lessening the severity or progression of one or more of nonalcoholic steatohepatitis (NASH), nonalcoholic fatty liver disease (NAFLD), cirrhosis of the liver, liver fibrosis, liver fibrogenesis, liver stiffness, liver inflammation or reducing one or more of immune cell infiltration, cytokine production in a liver, liver hydroxyproline levels, hepatic collagen production, body weight, fat mass, liver cholesterol, or liver lipids, in a subject in need thereof, comprising administering to the subject a therapeutically effective amount of a compound having the structure: 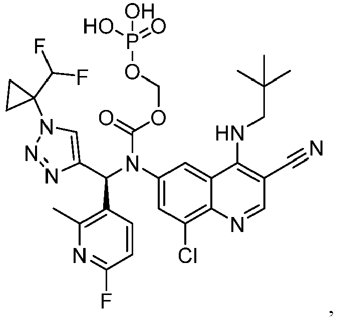 or a pharmaceutically acceptable salt thereof, in combination with an ACC inhibitor.

Described herein is a method of treating, stabilizing, or lessening the severity or progression of one or more of nonalcoholic steatohepatitis (NASH), nonalcoholic fatty liver disease (NAFLD), cirrhosis of the liver, liver fibrosis, liver fibrogenesis, liver stiffness, liver inflammation or reducing one or more of immune cell infiltration, cytokine production in a liver, liver hydroxyproline levels, hepatic collagen production, body weight, fat mass, liver cholesterol, or liver lipids, in a subject in need thereof, comprising administering to the subject a therapeutically effective amount of a compound having the structure: 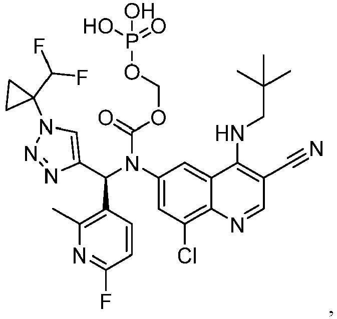 or a pharmaceutically acceptable salt thereof, in combination with firsocostat.

Described herein is a method of treating, stabilizing, or lessening the severity or progression of one or more of nonalcoholic steatohepatitis (NASH), nonalcoholic fatty liver disease (NAFLD), cirrhosis of the liver, liver fibrosis, liver fibrogenesis, liver stiffness, liver inflammation or reducing one or more of immune cell infiltration, cytokine production in a liver, liver hydroxyproline levels, hepatic collagen production, body weight, fat mass, liver cholesterol, or liver lipids, in a subject in need thereof, comprising administering to the subject a therapeutically effective amount of Compound A: 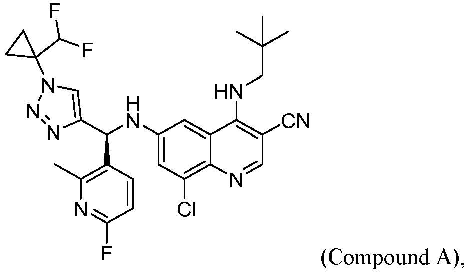 or a pharmaceutically acceptable salt thereof, in combination with Compound B: 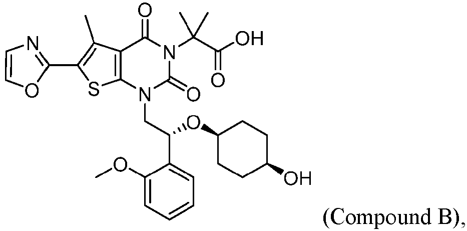 or a pharmaceutically acceptable salt thereof.

It will be appreciated, in embodiments where the compound which inhibits COT is administered with one or more additional therapeutic agents (e.g., an ACC inhibitor (ACCi)), the administration can occur on the same day or on different days and in any order as according to an appropriate dosing protocol.

In some embodiments, administration of a compound which inhibits COT starts a period of time (as such at least one day, three days, one week, two weeks, or a month) before administration of an ACC inhibitor starts, and optionally continues during at least part of the administration period of the ACC inhibitor. In some embodiments, administration of a compound which inhibits COT starts on the same day administration of an ACC inhibitor starts. In some embodiments, an ACC inhibitor is administered with a compound which inhibits COT for at least a period of time (as such at least one day, three days, one week, two weeks, or a month) after administration of the ACC inhibitor starts.

### Dosing of a compound which inhibits COT or the additional therapeutic agents

In some embodiments, a compound which inhibits COT or an optional additional therapeutic agent is administered in an amount of 0.1 mg/day to 1200 mg/day. In some embodiments, a compound which inhibits COT or each of the one or more optional additional agent is administered in an amount of 1 mg/day to 100 mg/day, 10 mg/day to 1200 mg/day, 10 mg/day to 100 mg/day, 100 mg/day to 1200 mg/day, 400 mg/day to 1200 mg/day, 600 mg/day to 1200 mg/day, 400 mg/day to 800 mg/day or 600 mg/day to 800 mg/day. In some embodiments, methods disclosed herein comprise the administration of 0.1 mg/day, 0.5 mg/day, 1 mg/day, 10 mg/day, 15 mg/day, 20 mg/day, 30 mg/day, 40 mg/day, 45 mg/day, 50 mg/day, 60 mg/day, 75 mg/day, 100 mg/day, 125 mg/day, 150 mg/day, 200 mg/day, 250 mg/day, 300 mg/day, 400 mg/day, 600 mg/day or 800 mg/day of a compound which inhibits COT and optionally a therapeutic agent to a subject in need thereof.

In some embodiments, the total daily dose of a compound which inhibits COT or each of the one or more additional therapeutic agents is selected from 5 mg, 10 mg, 20 mg, 25 mg, 30mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg, 500 mg, 510 mg, 520 mg, 530 mg, 540 mg, 550 mg, 560 mg, 570 mg, 580 mg, 590 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2050 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2700 mg, 2750 mg, 2800 mg, 2850 mg, 2900 mg, 2950 mg, or 3000 mg.

In some embodiments, the total daily dose of a compound which inhibits COT or each of the one or more additional therapeutic agents is independently between 5 mg to 3000 mg, between 5 mg to 1000 mg, between 5 mg to 500 mg, between 5 mg to 100 mg, between 10 mg to 3000 mg, between 10 mg to 2000 mg, between 10 mg to 1000 mg, between 20 mg to 1000 mg, between 30 mg to 1000 mg, between 30 mg to 750 mg, between 30 mg to 500 mg, between 30 mg to 250 mg, between 30 mg to 100 mg, between 50 mg to 500 mg, or between 50 mg to 100 mg.

### Unit Dosage Forms of additional therapeutic agents

In some embodiment, a compound which inhibits COT and each of the one or more optional additional therapeutic agents is administered in unit dosage formulations that comprise between 0.1 mg and 2000 mg, 1 mg and 200 mg, 35 mg and 1400 mg, 125 mg and 1000 mg, 250 mg and 1000 mg, or 500 mg and 1000 mg, or 100 mg and 1500 mg of the compound which inhibits COT or the one or more additional therapeutic agent.

In some embodiments, provided herein are unit dosage formulations comprising 0.1 mg, 0.25 mg, 0.5 mg, 1 mg, 5 mg, 10 mg, 15 mg, 20 mg, 30 mg, 45 mg, 50 mg, 60 mg, 75 mg, 100 mg, 125 mg, 150 mg, 200 mg, 250 mg, 300 mg, 400 mg, 600 mg or 800 mg of the compound which inhibits COT or the one or more additional therapeutic agent.

In some embodiments, provided herein are unit dosage formulations that comprise 0.1 mg, 0.25 mg, 0.5 mg, 1 mg, 2.5 mg, 5 mg, 10 mg, 15 mg, 20 mg, 30 mg, 35 mg, 50 mg, 70 mg, 100 mg, 125 mg, 140 mg, 175 mg, 200 mg, 250 mg, 280 mg, 350 mg, 500 mg, 560 mg, 700 mg, 750 mg, 1000 mg or 1400 mg of the compound which inhibits COT or the one or more additional therapeutic agent. In a particular embodiment, provided herein are unit dosage formulations that comprise 5 mg, 15 mg, 20 mg, 30 mg, 45 mg, and 50 mg of the compound which inhibits COT or the one or more additional therapeutic agent.

### Administration of a compound which inhibits COT or the one or more additional therapeutic agents

In some embodiments, the provided methods comprise administering a pharmaceutically acceptable composition comprising a compound which inhibits COT or the one or more additional therapeutic agent, one, two, three, or four times a day.

In some embodiments, a pharmaceutically acceptable composition comprising a compound which inhibits COT or the one or more additional therapeutic agent is administered once daily ("QD").

In some embodiments, a pharmaceutically acceptable composition comprising a compound which inhibits COT or the one or more additional therapeutic agent is administered twice daily. In some embodiments, twice daily administration refers to a compound or composition that is administered "BID", or two equivalent doses administered at two different times in one day.

In some embodiments, a pharmaceutically acceptable composition comprising a compound which inhibits COT or one or more additional therapeutic agents is administered three times a day. In some embodiments, a pharmaceutically acceptable composition comprising a compound which inhibits COT or one or more additional therapeutic agents is administered "TID", or three equivalent doses administered at three different times in one day.

In some embodiments, a pharmaceutically acceptable composition comprising a compound which inhibits COT or one or more additional therapeutic agents is administered four times a day. In some embodiments, a pharmaceutically acceptable composition comprising a compound which inhibits COT or one or more additional therapeutic agents is administered "QID", or four equivalent doses administered at four different times in one day. In some embodiments, a pharmaceutically acceptable composition comprising a compound which inhibits COT or one or more additional therapeutic agents is administered for a various number of days (for example 14, 21, 28) with a various number of days between treatment (0, 14, 21, 28).

In some embodiments, a compound which inhibits COT or an additional therapeutic agent are administered to a subject under fasted conditions and the total daily dose is any of those contemplated above and herein.

In some embodiments, a compound which inhibits COT or an additional therapeutic agent is administered to a subject under fed conditions and the total daily dose is any of those contemplated above and herein.

In some embodiments, a compound which inhibits COT or an additional therapeutic agent is administered orally. In some embodiments, when administered orally, a compound which inhibits COT or an additional therapeutic agent is administered with a meal and water. In another embodiment, a compound which inhibits COT or an additional therapeutic agent is dispersed in water or juice (e.g., apple juice or orange juice) and administered orally as a suspension. In some embodiments, when administered orally, a compound which inhibits COT or an additional therapeutic agent is administered in a fasted state.

A compound which inhibits COT or an additional therapeutic agent can also be administered intradermally, intramuscularly, intraperitoneally, percutaneously, intravenously, subcutaneously, intranasally, epidurally, sublingually, intracerebrally, intravaginally, transdermally, rectally, mucosally, by inhalation, or topically to the ears, nose, eyes, or skin. The mode of administration is left to the discretion of the health-care practitioner, and can depend in-part upon the site of the medical condition.

### Pharmaceutical Compositions and Modes of Administration

Compounds provided herein are usually administered in the form of pharmaceutical compositions. Thus, provided herein are also pharmaceutical compositions that contain one or more of the compounds described herein or a pharmaceutically acceptable salt, tautomer, stereoisomer, mixture of stereoisomers, or deuterated analog thereof and one or more pharmaceutically acceptable vehicles selected from carriers, adjuvants and excipients. Suitable pharmaceutically acceptable vehicles may include, for example, inert solid diluents and fillers, diluents, including sterile aqueous solution and various organic solvents, permeation enhancers, solubilizers and adjuvants. Such compositions are prepared in a manner well known in the pharmaceutical art. *See, e.g.,* Remington's Pharmaceutical Sciences, Mace Publishing Co., Philadelphia, Pa. 17th Ed. (1985); and Modern Pharmaceutics, Marcel Dekker, Inc. 3rd Ed. (G.S. Banker & C.T. Rhodes, Eds.).

As used herein, "pharmaceutically acceptable carrier" or "pharmaceutically acceptable excipient" includes any and all solvents, dispersion media, coatings, antibacterial and antifungal agents, isotonic and absorption delaying agents. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

The pharmaceutical compositions may be administered in either single or multiple doses. The pharmaceutical composition may be administered by various methods including, for example, rectal, buccal, intranasal and transdermal routes. In certain embodiments, the pharmaceutical composition may be administered by intra-arterial injection, intravenously, intraperitoneally, parenterally, intramuscularly, subcutaneously, orally, topically, or as an inhalant.

One mode for administration is parenteral, for example, by injection. The forms in which the pharmaceutical compositions described herein may be incorporated for administration by injection include, for example, aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Oral administration may be another route for administration of the compounds described herein. Administration may be via, for example, capsule or enteric coated tablets. In making the pharmaceutical compositions that include at least one compound described herein or a pharmaceutically acceptable salt, tautomer, stereoisomer, mixture of stereoisomers, or deuterated analog thereof, the active ingredient is usually diluted by an excipient and/or enclosed within such a carrier that can be in the form of a capsule, sachet, paper or other container. When the excipient serves as a diluent, it can be in the form of a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include lubricating agents such as talc, magnesium stearate, and mineral oil; wetting agents; emulsifying and suspending agents; preserving agents such as methyl and propylhydroxybenzoates; sweetening agents; and flavoring agents.

The compositions that include at least one compound described herein or a pharmaceutically acceptable salt, tautomer, stereoisomer, mixture of stereoisomers, or deuterated analog thereof can be formulated so as to provide quick, sustained or delayed release of the active ingredient after administration to the subject by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Patent Nos. 3,845,770; 4,326,525; 4,902,514; and 5,616,345. Another formulation for use in the methods disclosed herein employ transdermal delivery devices ("patches"). Such transdermal patches may be used to provide continuous or discontinuous infusion of the compounds described herein in controlled amounts. The construction and use of transdermal patches for the delivery of pharmaceutical agents is well known in the art. *See. e.g.,* U.S. Patent Nos. 5,023,252, 4,992,445 and 5,001,139. Such patches may be constructed for continuous, pulsatile, or on demand delivery of pharmaceutical agents.

For preparing solid compositions such as tablets, the principal active ingredient may be mixed with a pharmaceutical excipient to form a solid preformulation composition containing a homogeneous mixture of a compound described herein or a pharmaceutically acceptable salt, tautomer, stereoisomer, mixture of stereoisomers, or deuterated analog thereof. When referring to these preformulation compositions as homogeneous, the active ingredient may be dispersed evenly throughout the composition so that the composition may be readily subdivided into equally effective unit dosage forms such as tablets, pills and capsules.

The tablets or pills of the compounds described herein may be coated or otherwise compounded to provide a dosage form affording the advantage of prolonged action, or to protect from the acid conditions of the stomach. For example, the tablet or pill can include an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation may include solutions and suspensions in pharmaceutically acceptable, aqueous or organic solvents, or mixtures thereof, and powders. The liquid or solid compositions may contain suitable pharmaceutically acceptable excipients as described herein. In some embodiments, the compositions are administered by the oral or nasal respiratory route for local or systemic effect. In other embodiments, compositions in pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, preferably orally or nasally, from devices that deliver the formulation in an appropriate manner.

### Further Combination Therapies

In one embodiment, the compounds disclosed herein may be used in combination with one or more additional therapeutic agents. In some embodiments, a compound of the present disclosure, or a pharmaceutically acceptable salt thereof, can be combined with a therapeutically effective amount of one or more (*e*.*g*., one, two, three, four, one or two, one to three, or one to four) additional therapeutic agents.

In some embodiments, a compound of the disclosure, or a pharmaceutically acceptable salt thereof, is co-administered with one or more agents useful for the treatment and/or prophylaxis of an inflammatory, metabolic, and/or fibrotic condition or disease.

In some embodiments, a compound of the disclosure, or a pharmaceutically acceptable salt thereof, is co-administered with one or more agents useful for the treatment and/or prophylaxis of a heptatologic or nephrologic condition, such as NAFLD, NASH, DKD, or CKD. Non-limiting examples of such agents include metformin, sodium-glucose cotransporter-2 inhibitor (SGLT2i), drug therapy for glycemic control, DPP-4 inhibitor, insulin, sulfonylurea, TZD (thiazolidinedione), alpha-glucosidase inhibitor, SGLT2 inhibitor (e.g., empagliflozin, canagliflozin, dapaglifloz), glucagon-like peptide-1 receptor agonist (GLP-1 RA) (e.g., lixisenatide, liraglutide, semaglutide, exenatide, albiglutide, dulaglutide), DPP-4 inhibitors (e.g., saxagliptin, alogliptin, sitagliptin, linagliptin), one or more agents used to treat high blood pressure such as angiotensin-converting enzyme (ACE) inhibitors and angiotensin 2 receptor blockers (ARBs), agents supportive of weight loss or for control of blood sugar, cholesterol-lowering drugs (e.g., statins), finerenone, and agents for treatment of diabetes mellitus, such as alpha-glucosidase inhibitors (e.g., acarbose, miglitol, voglibose).

In some embodiments, a compound of the disclosure, or a pharmaceutically acceptable salt thereof, is co-administered with one or more agents useful for the treatment and/or prophylaxis of a gastroenterologic condition such as ulcerative colitis (UC) or Crohn's disease (CD). Non-limiting examples of such agents include infliximab, adalimumab, golimumab, vedolizumab, tofacitinib, ustekinumab, natalizumab, mesalamine, diazo-bonded 5-ASA, sulfasalazine, balsalazide, olsalazine, corticosteroids such as budesonide, hydrocortisone, methylprednisolone, and prednisone; immunosuppressants or immunomodulators such as azathioprine and 6-mercaptopurine, cyclosporine, and methotrexate.

In some embodiments, the additional therapeutic agent comprises an apoptotic signal-regulating kinase (ASK-1) inhibitor, a farnesoid X receptor (FXR) agonist, a peroxisome proliferator-activated receptor alpha (PPARα) agonist, fish oil, an acetyl-coA carboxylase (ACC) inhibitor, a TGFβ antagonist, a LPAR antagonist, a SGLT2 inhibitor, a Tpl2 inhibitor, a GLP-1 agonist, or a combination thereof.

The benefit of combination may be increased efficacy and/or reduced side effects for a component as the dose of that component may be adjusted down to reduce its side effects while benefiting from its efficacy augmented by the efficacy of the compound of the present disclosure.

In some embodiments, the therapeutic agent, or combination of therapeutic agents, are a(n) ACE inhibitor, 2-Acylglycerol O-acyltransferase 2 (DGAT2) inhibitor, Acetaldehyde dehydrogenase inhibitor, Acetyl CoA carboxylase inhibitor, Adrenergic receptor agonist, Alstrom syndrome protein 1(ALMS1)/PKC alpha protein interaction inhibitor, Apelin receptor agonist, Diacylglycerol O acyltransferase 2 inhibitor, Adenosine A3 receptor agonist, Adenosine A3 receptor antagonist, Adiponectin receptor agonist, Aldehyde dehydrogenase 2 stimulator, AKT protein kinase inhibitor, AMP-activated protein kinases (AMPK), AMP kinase activator, ATP citrate lyase inhibitor, AMP activated protein kinase stimulator, Endothelial nitric oxide synthase stimulator, NAD-dependent deacetylase sirtuin-1 stimulator, Adrenergic receptor antagonist, Androgen receptor agonist, Amylin receptor agonist, Angiotensin II AT-1 receptor antagonist, Apical sodium-dependent bile acid transport inhibitor, Autophagy protein modulator, Autotaxin inhibitors, Axl tyrosine kinase receptor inhibitor, Bax protein stimulator, Beta-catenin inhibitor, Bioactive lipid, Calcitonin agonist, Cannabinoid receptor modulator, Caspase inhibitor, Caspase-3 stimulator, Cathepsin inhibitor, Caveolin 1 inhibitor, CCK receptor antagonist, CCL26 gene inhibitor, CCR2 chemokine antagonist, CCR2 chemokine antagonist, Angiotensin II AT-1 receptor antagonist, CCR3 chemokine antagonist, CCR5 chemokine antagonist, CD3 antagonist, CDGSH iron sulfur domain protein modulator, chitinase inhibitor, Chloride channel stimulator, Chitotriosidase 1 inhibitor, CNR1 inhibitor, Connective tissue growth factor ligand inhibitor, COT protein kinase inhibitor, Cyclin D1 inhibitor, Cytochrome P450 7A1 inhibitor, Cytochrome P450 reductase inhibitors, DGAT1/2 inhibitor, Diacylglycerol O acyltransferase 1 inhibitor (DGAT1), Cytochrome P450 2E1 inhibitor (CYP2E1), CXCR3 chemokine antagonist, CXCR4 chemokine antagonist, Dihydroceramide delta 4 desaturase inhibitor, Dihydroorotate dehydrogenase inhibitor, Dipeptidyl peptidase IV inhibitor, Endosialin modulator, Eotaxin ligand inhibitor, Extracellular matrix protein modulator, Farnesoid X receptor agonist, Fatty acid synthase inhibitors, FGF1 receptor agonist, Fibroblast growth factor (FGF-15, FGF-19, FGF-21) ligands, fibroblast activation protein inhibitor, Free fatty acid receptor 1 agonist, Galectin-3 inhibitor, GDNF family receptor alpha like agonist, Glucagon receptor agonist, Glucagon-like peptide 1 agonist, Glucocorticoid receptor antagonist, Glucose 6-phosphate 1-dehydrogenase inhibitor, G-protein coupled bile acid receptor 1 agonist, G-protein coupled receptor-119 agonist, G-protein coupled receptor 84 antagonist, Hedgehog (Hh) modulator, Hepatitis C virus NS3 protease inhibitor, Hepatocyte nuclear factor 4 alpha modulator (HNF4A), Hepatocyte growth factor modulator, Histone deacetylase inhibitor, STAT-3 modulator, HMG CoA reductase inhibitor, HSD17B13 gene inhibitor, 5-HT 2a receptor antagonist, Hydrolase inhibitor, Hypoxia inducible factor-2 alpha inhibitor, IL-10 agonist, IL-17 antagonist, IL-22 agonist, Ileal sodium bile acid cotransporter inhibitor, Insulin sensitizer, Insulin ligand agonist, Insulin receptor agonist, integrin modulator, Integrin Antagonist, Integrin alpha-V/beta-1 antagonist, Integrin alpha-V/beta-6 antagonist, intereukin-1 receptor-associated kinase 4 (IRAK4) inhibitor, IL-6 receptor agonist, interleukin 17 ligand inhibitor, Jak2 tyrosine kinase inhibitor, Jun N terminal kinase-1 inhibitor, Kelch like ECH associated protein 1 modulator, Ketohexokinase (KHK) inhibitor, Klotho beta stimulator, Leukotriene A4 hydrolase inhibitor, 5-Lipoxygenase inhibitor, Lipoprotein lipase inhibitor, Liver X receptor, LPL gene stimulator, Lysophosphatidate-1 receptor antagonist, Lysyl oxidase homolog 2 inhibitor, LXR inverse agonists, Macrophage mannose receptor 1 modulator, Matrix metalloproteinases (MMPs) inhibitor, MEKK-5 protein kinase inhibitor, MCH receptor-1 antagonist, Membrane copper amine oxidase (VAP-1) inhibitor, Methionine aminopeptidase-2 inhibitor, Methyl CpG binding protein 2 modulator, MicroRNA-132 (miR-132) antagonist, MicroRNA-21(miR-21) inhibitor, Mitochondrial uncoupler, Mixed lineage kinase-3 inhibitor, Motile sperm domain protein 2 inhibitor, Myelin basic protein stimulator, NACHT LRR PYD domain protein 3 (NLRP3) inhibitor, NAD-dependent deacetylase sirtuin stimulator, NADPH oxidase inhibitor (NOX), NFE2L2 gene inhibitor, Nicotinic acid receptor 1 agonist, Opioid receptor mu antagonist, P2Y13 purinoceptor stimulator, Nuclear erythroid 2-related factor 2 stimulator, Nuclear receptor modulators, Nuclear transport of transcription factor modulator, P2X7 purinoceptor modulator, PACAP type I receptor agonist, PDE 3 inhibitor, PDE 4 inhibitor, PDE 5 inhibitor, PDGF receptor beta modulator, Phenylalanine hydroxylase stimulator, Phospholipase C inhibitor, Phosphoric diester hydrolase inhibitor, PPAR alpha agonist, PPAR delta agonist, PPAR gamma agonist, Peptidyl-prolyl cis-trans isomerase A inhibitor, PNPLA3 gene inhibitor, PPAR gamma modulator, Protease-activated receptor-2 antagonist, Protein kinase modulator, Protein NOV homolog modulator, PTGS2 gene inhibitor, renin inhibitor, Resistin/CAP1 (adenylyl cyclase associated protein 1) interaction inhibitor, Rho associated protein kinase inhibitor, RNA polymerase inhibitors, S-nitrosoglutathione reductase (GSNOR) enzyme inhibitor, Sodium glucose transporter-2 inhibitor, Sphingolipid delta 4 desaturase DES1 inhibitor, SREBP transcription factor inhibitor, STAT-1 inhibitor, Stearoyl CoA desaturase-1 inhibitor, STK25 inhibitor, Suppressor of cytokine signalling-1 stimulator, Suppressor of cytokine signalling-3 stimulator, Taste receptor type 2 agonist, Telomerase stimulator, TERT gene modulator, TGF beta (TGFB1) ligand inhibitor, TNF antagonist, Transforming growth factor β (TGF-β), Transforming growth factor β activated Kinase 1 (TAK1), Thyroid hormone receptor beta agonist, TLR-4 antagonist, Transglutaminase inhibitor, Tyrosine kinase receptor modulator, GPCR modulator, nuclear hormone receptor modulator, TLR-9 antagonist, VDR agonist, Vitamin D3 receptor modulators, WNT modulators, YAP/TAZ modulator or a Zonulin inhibitor, and combinations thereof.

Non-limiting examples of the one or more additional therapeutic agents include:
ACE inhibitors, such as enalapril;
Acetaldehyde dehydrogenase inhibitors, such as ADX-629;
Acetyl CoA carboxylase (ACC) inhibitors, such as NDI-010976 (firsocostat), DRM-01, gemcabene, GS-834356, PF-05175157, QLT-091382, PF-05221304;
Acetyl CoA carboxylase/Diacylglycerol O acyltransferase 2 inhibitors, such as PF-07055341;
Adenosine receptor agonists, such as CF-102 (namodenoson), CF-101 (piclidenoson), CF-502, CGS21680;
Adenosine A3 receptor antagonist, such as FM-101;
Adiponectin receptor agonists, such as ADP-355, ADP-399, ALY668-SR;
Adrenergic receptor antagonist, such as bromocriptine, phentermine, VI-0521;
Aldehyde dehydrogenase 2 stimulators, such as FP-045;
Amylin/calcitonin receptor agonists, such as KBP-042, KBP-089;
AMP activated protein kinase stimulators, such as C-455, PXL-770, O-304;
AMP kinase activators/ATP citrate lyase inhibitors, such as bempedoic acid (ETC-1002, ESP-55016);
AMP activated protein kinase/Endothelial nitric oxide synthase/NAD-dependent deacetylase sirtuin-1 stimulators, such as NS-0200 (leucine + metformin + sildenafil);
Androgen receptor agonists, such as LPCN-1144, LPCN-1148, testosterone prodrug;
Angiotensin II AT-1 receptor antagonists, such as irbesartan; Angiopoietin-related protein-3 inhibitors, such as vupanorsen (IONIS-ANGPTL3-LRx);
Apelin receptor agonist, such as CB-5064, MBT-2;
Apical sodium-dependent bile acid transport inhibitors, such as A-3907;
Autophagy protein modulators, such as A-2906, GM-90194;
Autotaxin (ectonucleotide pyrophosphatase/phosphodiesterase 2 (NPP2 or ENPP2)) inhibitors, such as FP10.47, PAT-505, PAT-048, GLPG-1690, X-165, PF-8380, TJC-0265, TJC-0316, AM-063, BBT-877;
Axl tyrosine kinase receptor inhibitors, such as bemcentinib (BGB-324, R-428);
Bax protein stimulators, such as CBL-514;
Bioactive lipids, such as DS-102;
Cannabinoid receptor modulators, such as namacizumab (nimacimab), GWP-42004, REV-200, CRB-4001, INV-101, SCN-002;
Caspase inhibitors, such as emricasan;
Pan cathepsin B inhibitors, such as VBY-376;
Pan cathepsin inhibitors, such as VBY-825;
CCK receptor antagonist, such as proglumide;
CCL26 gene inhibitor, such as mosedipimod, KDDF-201410-10;
CCR2/CCR5 chemokine antagonists, such as BMS-687681, cenicriviroc, maraviroc, CCX-872, leronlimab, WXSH-0213;
CCR2/CCR5 chemokine antagonists and FXR agonists, such as LJC-242 (tropifexor + cenivriviroc);
CCR2 chemokine antagonists, such as propagermanium;
CCR2 chemokine/Angiotensin II AT-1 receptor antagonists, such as DMX-200, DMX-250;
CCR3 chemokine antagonists, such as bertilimumab;
CD3 antagonists, such as NI-0401 (foralumab);
CDGSH iron sulfur domain protein modulators, such as EYP-002;
Chitinase inhibitor, such as OATD-01;
Chitotriosidase 1 inhibitors, such as OAT-2068;
Chloride channel stimulators, such as cobiprostone, and lubiprostone;
Casein kinase-1 (CK1) delta/epsilon inhibitors, such as PF-05006739;
Connective tissue growth factor ligand inhibitor, such as PBI-4050;
COT protein kinase inhibitors, such as GS-4875, GS-5290;
CXCR4 chemokine antagonists, such as AD-214;
Cytochrome P450 reductase inhibitors, such as SNP-630;
Diglyceride acyltransferase 2 (DGAT2) inhibitors, such as IONIS-DGAT2Rx, PF-06865571;
Diglyceride acyltransferase 1 (DGAT1) inhibitors, such as GSK-3008356;
Diacylglycerol O acyltransferase 1 (DGAT1)/ Cytochrome P450 2E1 inhibitors (CYP2E1), such as SNP-610;
Dihydroorotate dehydrogenase inhibitor, such as vidofludimus;
Dipeptidyl peptidase IV inhibitors, such as linagliptin,evogliptin;
Eotaxin ligand inhibitors, such as bertilimumab, CM-101;
Extracellular matrix protein modulators, such as CNX-024;
Farnesoid X receptor (FXR) agonists, such as AGN-242266, AGN-242256, ASC-42, EDP-297 (EP-024297), RDX-023, BWL-200, AKN-083, EDP-305, GNF-5120, cilofexortromethamine (GS-9674), HPG-1860, IOT-022, LMB-763, obeticholic acid, Px-102, Px-103, M790, M780, M450, M-480, MET-409, MET-642, PX20606, SYHA-1805, vonafexor (EYP-001), TERN-101, TC-100, INT-2228, TQA-3526, ZG-5266, HPD-001, alendronate;
Farnesoid X receptor (FXR)/ G-protein coupled bile acid receptor 1(TGR5) agonists, such as INT-767;
Fatty acid synthase inhibitors, such as TVB-2640, FT-8225;
Fibroblast growth factor 19 (rhFGF19)/cytochrome P450 (CYP) 7A1 inhibitors, such as aldafermin (NGM-282);
Fibroblast growth factor 21(FGF-21) ligand modulators, such as AP-025, BMS-986171, B-1654, BIO89-100, BOS-580, Pegbelfermin (BMS-986036), B-1344, NN-9499;
Fibroblast growth factor 21 (FGF-21)/glucagon like peptide 1 (GLP-1) agonists, such as YH-25723 (YH-25724; YH-22241), efruxifermin (AKR-001);
FGF receptor agonists/Klotho beta stimulators, such as BFKB-8488A (RG-7992);
Free fatty acid receptor 1 agonist, such as SCO-267;
Galectin-3 inhibitors, such as belapectin (GR-MD-02), GB-1107 (Gal-300), GB-1211 (Gal-400), IMT-001;
GDNF family receptor alpha like agonist, such as NGM-395;
Glucagon-like peptide 1 (GLP1R) agonists, such as ALT-801, AC-3174, liraglutide, cotadutide (MEDI-0382), SAR-425899, LY-3305677, HM-15211, YH-25723, YH-GLP1, RPC-8844, PB-718, PF-06882961, semaglutide;
Glucagon-like peptide 1 receptor agonist; Oxyntomodulin ligand; Glucagon receptor agonist, such as efinopegdutide;
Gastric inhibitory polypeptide/Glucagon-like peptide-1 (GIP/GLP-1) receptor co-agonist, such as tirzepatide (LY-3298176);
PEGylated long-acting glucagon-like peptide-1/glucagon (GLP-1R/GCGR) receptor dual agonist, such as DD-01;
Glucagon/GLP1-receptor agonist, such as BI-456906, NN-6177;
Glucocorticoid receptor antagonists, such as CORT-118335 (miricorilant);
Glucose 6-phosphate 1-dehydrogenase inhibitors, such as ST001;
Glucokinase stimulator, such as dorzagliatin, sinogliatin (RO-5305552);
G-protein coupled bile acid receptor 1(TGR5) agonists, such as RDX-009, INT-777, HY-209;
G-protein coupled receptor 84 antagonist, such as PBI-4547;
G-protein coupled receptor-119 agonist, such as DA-1241;
Heat shock protein 47 (HSP47) inhibitors, such as ND-L02-s0201;
Hedgehog protein TGF beta ligand inhibitors, such as Oxy-210;
Histone deacetylase inhibitors/ STAT-3 modulators, such as SFX-01;
HMG CoA reductase inhibitors, such as atorvastatin, fluvastatin, pitavastatin, pravastatin, rosuvastatin, simvastatin;
HSD17B13 gene inhibitor, such as ALN-HSD, ARO-HSD;
Hydrolase inhibitor, such as ABD-X;
Hypoxia inducible factor-2 alpha inhibitors, such as PT-2567;
IL-10 agonists, such as peg-ilodecakin;
Ileal sodium bile acid cotransporter inhibitors, such as odevixibat (A-4250), volixibat potassium ethanolate hydrate (SHP-262), GSK2330672, CJ-14199, elobixibat (A-3309);
Insulin sensitizers, such as, KBP-042, azemiglitazone potassium (MSDC-0602K), ION-224, MSDC-5514, Px-102, RG-125 (AZD4076), Tolimidone, VVP-100X, CB-4211, ETI-101;
Insulin ligand/dsInsulin receptor agonists, such as ORMD-0801;
Integrin antagonists, such as IDL-2965;
IL-6 receptor agonists, such as KM-2702;
Integrin alpha-V/beta-6 and alpha-V/beta-1 dual inhibitor; such as PLN-74809;
Interleukin 17 ligand inhibitor, such as netakimab;
Jak1/2 tyrosine kinase inhibitor, such as baricitinib;
Jun N terminal kinase-1 inhibitor, such as CC-90001;
Kelch like ECH associated protein 1 modulator, such as alpha-cyclodextrin-stabilized sulforaphane;
Ketohexokinase (KHK) inhibitors, such as PF-06835919, LY-3478045, LY-3522348;
beta Klotho (KLB)- FGF1c agonists, such as MK-3655 (NGM-313);
Leukotriene A4 hydrolase inhibitor, such as LYS-006;
5-Lipoxygenase inhibitors, such as tipelukast (MN-001), epeleuton (DS-102, (AF-102);
Lipoprotein lipase inhibitors, such as CAT-2003;
LPL gene stimulators, such as alipogene tiparvovec;
Liver X receptor (LXR) inhibitors, such as PX-665, PX-L603, PX-L493, BMS-852927, T-0901317, GW-3965, SR-9238;
Lysophosphatidate-1 receptor antagonists, such as BMT-053011, UD-009 (CP-2090), AR-479, ITMN-10534, BMS-986020, KI-16198, BMS-986278, BMS-986234;
Lysyl oxidase homolog 2 inhibitors, such as simtuzumab, PXS-5382A (PXS-5338);
Macrophage mannose receptor 1 modulators, such as tilmanocept-Cy3 (technetium Tc 99m tilmanocept);
Matrix metalloprotease inhibitors, such as ALS-L1023;
Membrane copper amine oxidase (VAP-1) inhibitors, such as TERN-201, TT-01025;
MEKK-5 protein kinase (ASK-1) inhibitors, such as CJ-16871, CS-17919, selonsertib (GS-4997), SRT-015, GS-444217, GST-HG-151, TERN-301;
MCH receptor-1 antagonists, such as CSTI-100 (ALB-127158);
Semicarbazide-Sensitive Amine Oxidase/Vascular Adhesion Protein-1 (SSAO/VAP-1) Inhibitors, such as PXS-4728A (BI-1467335);
Methionine aminopeptidase-2 inhibitors, such as ZGN-1061, ZGN-839, ZN-1345;
Methyl CpG binding protein 2 modulators, such as mercaptamine;
Mineralocorticoid receptor antagonists (MCRA), such as MT-3995 (apararenone);
Mitochondrial uncouplers, such as 2,4-dinitrophenol, HU6, Mito-99-0053;
Mixed lineage kinase-3 inhibitors, such as URMC-099-C;
Motile sperm domain protein 2 inhibitors, such as VB-601;
Myelin basic protein stimulators, such as olesoxime;
Myeloperoxidase inhibitors, such as PF-06667272, AZM-198;
NADPH oxidase inhibitors, such as GKT-831, GenKyoTex, APX-311, setanaxib;
Nicotinic acid receptor 1 agonists, such as ARI-3037MO;
NACHT LRR PYD domain protein 3 (NLRP3) inhibitors, such as KDDF-201406-03, NBC-6, IFM-514, JT-194 (JT-349);
NFE2L2 gene inhibitor, such as GeRP-amiR-144;
Nuclear transport of transcription factor modulators, such as AMTX-100;
Nuclear receptor modulators, such as DUR-928 (DV-928);
Opioid receptor mu antagonists, such as methylnaltrexone;
P2X7 purinoceptor modulators, such as SGM-1019;
P2Y13 purinoceptor stimulators, such as CER-209;
PDE 3/4 inhibitors, such as tipelukast (MN-001);
PDE 5 inhibitors, such as sildenafil, MSTM-102;
PDGF receptor beta modulators, such as BOT-191, BOT-509;
Peptidyl-prolyl cis-trans isomerase inhibitors, such as CRV-431 (CPI-432-32), NVP-018, NV-556 (NVP-025);
Phenylalanine hydroxylase stimulators, such as HepaStem;
Phosphoric diester hydrolase inhibitor, such as ZSP-1601;
PNPLA3 gene inhibitor, such as AZD-2693;
PPAR agonists, such as Chiglitazar, elafibranor (GFT-505), seladelpar lysine (MBX-8025), deuterated pioglitazone R-enantiomer, pioglitazone, PXL-065 (DRX-065), saroglitazar, lanifibranor (IVA-337), CHS-131, pemafibrate (K-877), ZG-0588, ZSP-0678; ZSYM-008; fenofibrate;
Protease-activated receptor-2 antagonists, such as PZ-235;
Protein kinase modulators, such as CNX-014;
Protein NOV homolog modulators, such as BLR-200;
PTGS2 gene inhibitors, such as STP-705, STP-707;
Renin inhibitors, such as PRO-20;
Resistin/CAP1 (adenylyl cyclase associated protein 1) interaction inhibitors, such as DWJ-211;
Rev protein modulator, such as ABX-464;
Rho associated protein kinase (ROCK) inhibitors, such as REDX-10178 (REDX-10325), KD-025, RXC-007, TDI-01;
RNA polymerase inhibitors, such as rifaximin;
Snitrosoglutathione reductase (GSNOR) enzyme inhibitors, such as SL-891;
Sodium glucose transporter-2 (SGLT2) inhibitors, such as ipragliflozin, remogliflozin etabonate, ertugliflozin, dapagliflozin, tofogliflozin, sotagliflozin;
Sodium glucose transporter-1/2 (SGLT 1/2) inhibitors, such as licogliflozin bis(prolinate) (LIK-066);
SREBP transcription factor inhibitors, such as CAT-2003, HPN-01, MDV-4463;
Stearoyl CoA desaturase-1 inhibitors, such as aramchol;
Taste receptor type 2 agonists, such as ARD-101;
Thyroid hormone receptor beta agonists, such as ALG-009, ASC-41, CNPT-101101; CNPT-101207, CS-27186, KY-41111, resmetirom (MGL-3196), MGL-3745, TERN-501, VK-2809, HP-515;
TLR-2/TLR-4 antagonists, such as VB-201 (CI-201);
TLR-4 antagonists, such as JKB-121, JKB-122, naltrexone;
Tyrosine kinase receptor modulators, such as CNX-025, GFE-2137 (repurposed nitazoxanide);
TLR-9 antagonist, such as GNKS-356, AVO-101;
TNF antagonist, such as ALF-421;
GPCR modulators, such as CNX-023;
Nuclear hormone receptor modulators, such as Px-102;
VDR agonist, such as CK-15;
Xanthine oxidase inhibitors, such as ACQT-1127;
Xanthine oxidase/Urate anion exchanger 1 (URAT1) inhibitors, such as RLBN-1001, RLBN-1127; or
Zonulin Inhibitors, such as larazotide acetate (INN-202).

In certain specific embodiments, the one or more additional therapeutic agents are selected from A-4250, AC-3174, acetylsalicylic acid, AK-20, alipogene tiparvovec, AMX-342, AN-3015, anti-CXCR3 antibodies, anti-TAGE antibody, aramchol, ARI-3037MO, ASP-8232, AXA-1125, bertilimumab, Betaine anhydrous, BI-1467335, BMS-986036, BMS-986171, BMT-053011, BOT-191, BTT-1023, budesonide, BX-003, CAT-2003, cenicriviroc, CBW-511, CER-209, CF-102, CGS21680, CNX-014, CNX-023, CNX-024, CNX-025, cobiprostone, colesevelam, dabigatran etexilate mesylate, dapagliflozin, DCR-LIV1, deuterated pioglitazone R-enantiomer, 2,4-dinitrophenol, DRX-065, DS-102, DUR-928, edaravone (TTYP-01), EDP-305, elafibranor (GFT-505), emricasan, enalapril, ertugliflozin, evogliptin, F-351, fluasterone (ST-002), FT-4101, GDD-3898, GH-509, GKT-831, GNF-5120, GRI-0621, GR-MD-02, GS-300, GS-4997, GS-9674, GS-4875, GS-5290, HEC-96719, HTD-1801, HS-10356, HSG-4112, HST-202, HST-201, HU-6, hydrochlorothiazide, icosabutate (PRC-4016), icosapent ethyl, IMM-124-E, INT-767, INV-240, ION-455, IONIS-DGAT2Rx, ipragliflozin, Irbesarta, propagermanium, IVA-337, J2H-1702, JKB-121, KB-GE-001, KBLP-004, KBLP-009, KBP-042, KD-025, M790, M780, M450, metformin, sildenafil, LB-700, LC-280126, linagliptin, liraglutide, (LJN-452) (tropifexor), LM-011, LM-002 (CVI-LM-002), LMB-763, LYN-100, MB-N-008, MBX-8025, MDV-4463, mercaptamine, MGL-3196, MGL-3745, MP-301, MSDC-0602K, namacizumab, NC-101, NDI-010976, ND-L02-s0201 (BMS-986263), NGM-282, NGM-313, NGM-386, NGM-395, NP-011, NP-135, NP-160, norursodeoxycholic acid, NV-422, NVP-022, 0-304, obeticholic acid (OCA), 25HC3S, olesoxime, PAT-505, PAT-048, PBI-4547, peg-ilodecakin, pioglitazone, pirfenidone, PRI-724, PX20606, Px-102, PX-L603, PX-L493, PXS-4728A, PZ-235, PZH-2109, RCYM-001, RDX-009, remogliflozin etabonate, RG-125 (AZD4076), RP-005, RPI-500, S-723595, saroglitazar, SBP-301, semaglutide, SH-2442, SHC-028, SHC-023, simtuzumab, solithromycin, sotagliflozin, statins (atorvastatin, fluvastatin, pitavastatin, pravastatin, rosuvastatin, simvastatin), TCM-606F, TEV-45478, TQA-3526, TQA-3563, tipelukast (MN-001), TLY-012, TRX-318, TVB-2640, TXR-611, TXR-612, TS-20004, UD-009, UN-03, ursodeoxycholic acid, VBY-376, VBY-825, VK-2809, vismodegib, volixibat potassium ethanolate hydrate (SHP-626), VVP-100X, WAV-301, WNT-974, WXSH-0038, WXSH-0078, XEN-103, XRx-117, XTYW-003, XW-003, XW-004, XZP-5610, ZGN-839, ZG-5216, ZSYM-008, or ZYSM-007.

Examples of Acetyl CoA carboxylase (ACC) inhibitors include those described in US2013123231, US2019134041, US2017267690, US2018298025.

Examples of Acetyl CoA carboxylase (ACC) inhibitors/Farnesoid X receptor (FXR) agonists include those described in US2018280394.

Examples of Acetyl CoA carboxylase (ACC) inhibitors/Farnesoid X receptor (FXR) agonists/MEKK-5 protein kinase (ASK-1) inhibitors include those described in US2018021341, US2018333401.

Examples of Acetyl CoA carboxylase (ACC)/ MEKK-5 protein kinase (ASK-1) inhibitors include those described in US2018311244.

Examples of Farnesoid X receptor (FXR) agonists include those described in US2014221659, US2020281911, WO2020185685.

Examples of Farnesoid X receptor (FXR) agonists/MEKK-5 protein kinase (ASK-1) inhibitors include those described in US2017273952, US201813320.

Examples of MEKK-5 protein kinase (ASK-1) inhibitors include those described in US2011009410, US2013197037, US2016244430, US2016280683.

### Kits

Provided herein are also kits that include a compound described herein, or a pharmaceutically acceptable salt, tautomer, stereoisomer, mixture of stereoisomers, or deuterated analog thereof, and suitable packaging. In one embodiment, a kit further includes instructions for use. In one aspect, a kit includes a compound of Formula I (or any other Formula described herein), or a pharmaceutically acceptable salt, tautomer, stereoisomer, mixture of stereoisomers, or deuterated analog thereof, and a label and/or instructions for use of the compounds in the treatment of the indications, including the diseases or conditions, described herein.

Provided herein are also articles of manufacture that include a compound described herein or a pharmaceutically acceptable salt, tautomer, stereoisomer, mixture of stereoisomers, or deuterated analog thereof in a suitable container. The container may be a vial, jar, ampoule, preloaded syringe, and intravenous bag.

### Examples

The following examples are included to demonstrate specific embodiments of the disclosure. It should be appreciated by those of skill in the art that the techniques disclosed in the examples which follow represent techniques to function well in the practice of the disclosure, and thus can be considered to constitute specific modes for its practice. However, those of skill in the art should, in light of the present disclosure, appreciate that many changes can be made in the specific embodiments which are disclosed.

In the examples below, Compound A has the structure: 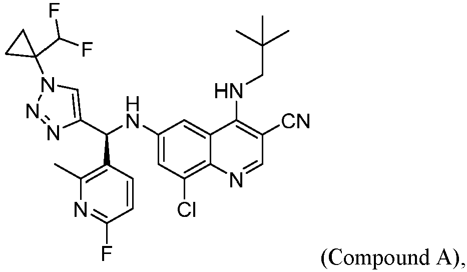 which can be obtained according to the procedure described in WO2017007689A1 (see, Compound 447 and Procedure 27 in WO2017007689A1), and Compound B has the structure: 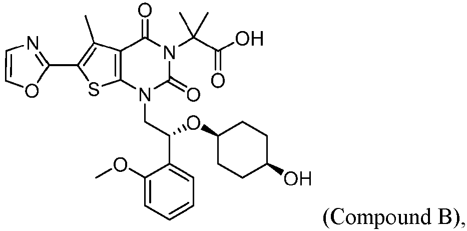 which can be obtained according to the procedure described in U.S. 9,453,026 (see, Compound I-278 and Example 128 in U.S. 9,453,026). Cenicriviroc (CVC) was purchased from commercial sources (ACCEL pharmatech).

### Example 1: Pharmacology Study with Compound A and Compound B Using the Choline Deficient High Fat Diet Model of Fatty Liver in the Male Wistar Han Rats

Compound A and Compound B were studied in a Choline Deficient High Fat Diet (CDHFD) model as described below. Rats enrolled in this study were first fed a choline deficient high fat diet (CDHFD) for 42 days prior to test article administration. Animals were then split into treatment groups 1-8 and test article was administered as detailed in Table 1 for 42 days.

**Table 1: Test Details**

| **Treatment group/ Diet** | **No. of animals** | **Test article** | **Dose mg/Kg** | **Dose Route** | **Regimen** | **Dose Vol (mL/Kg)** | **Dose (mg/mL)** |
|---|---|---|---|---|---|---|---|
| 1/ Chow | 10 | Vehicle | 0 | PO | BID | 5 | 0 |
| 2/ CDHFD | 15 | Vehicle | 0 | PO | BID | 5 | 0 |
| 3/ CDHFD | 15 | Compound A | 100 | PO | QD | 5 | 20 |
| 4/ CDHFD | 15 | Compound A | 100 | PO | BID | 5 | 20 |
| 5/ CDHFD | 15 | Compound A + Compound B | 100 + 10 | PO | QD | 2.5 + 2.5 | 20 + 4 |
| 6/ CDHFD | 15 | Compound A + Compound B | 100 + 10 | PO | BID + QD | 2.5 + 2.5 | 20 + 4 |
| 7/ CDHFD | 15 | Compound B | 10 | PO | QD | 5 | 2 |
| 8/ CDHFD | 10 | Diet Control | N/A | N/A | N/A | N/A | N/A |

| | | | | | | | |
|---|---|---|---|---|---|---|---|
| • NA = Not Applicable • CDHFD diet: A16092003O from Research Diets with orange dye. Chow diet: Teklad Global Diets - Rodent 8640 (Standard Diet). • Vehicle: Copovidone and TPGS Vehicle (5% D-α-tocopherol polyethylene glycol succinate (TPGS), 10% copovidone (PVP-VA), 85% 0.01M HCl in deionized water (pH 2 water)). • Livers were collected at termination. | | | | | | | |

Liver tissue obtained at the end of the study was subjected to picrosirius red staining (PSR) was assessed according to standard techniques. Liver hydroxyproline content was measured from cryopowdered liver tissue. Chloramine-T was used to oxidize the free hydroxyproline for the production of a pyrrole. Addition of Ehrlich's reagent results in the formation of a chromophore that can be measured at 550 nm on a spectramax plate reader.

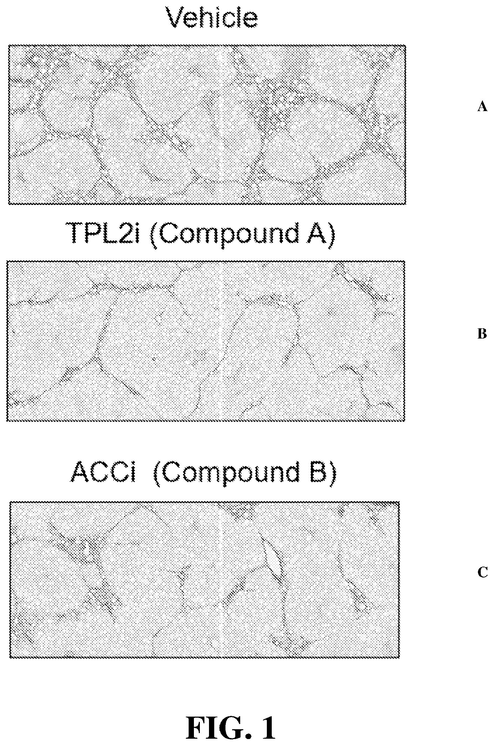

**FIG. 1****,** panel A, shows liver tissue with picrosirius red staining (PSR) from animals in treatment group 2 which were administered vehicle. **FIG. 1****,** panel B, shows liver tissue with picrosirius red staining (PSR) from animals in treatment group 4 which were administered a COT inhibitor (Compound A). **FIG. 1****,** panel C, shows liver tissue with picrosirius red staining (PSR) from animals in treatment group 7 which were administered an ACC inhibitor (Compound B). **FIG. 1****,** panels A, B and C, show that the COT inhibitor (Compound A) treated animal exhibited less liver fibrosis as compared to animals treated with vehicle.

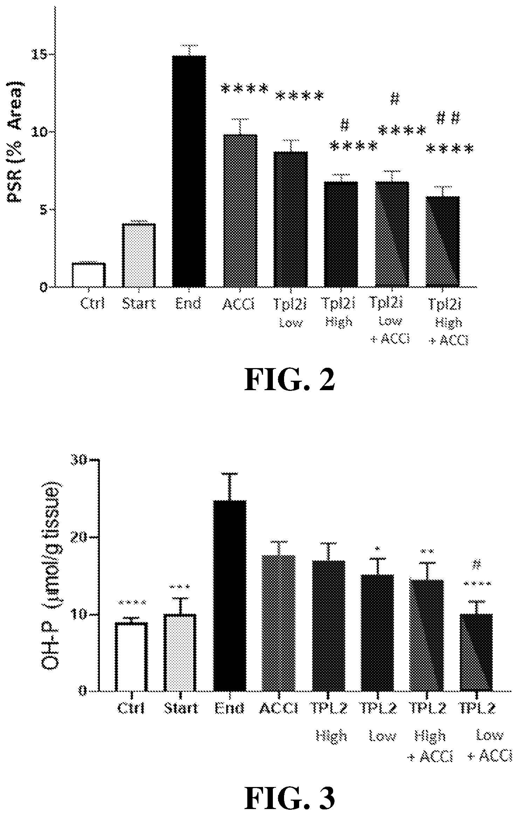

**FIG. 2** plots the PSR (% area) for control (treatment group 1), start and end for the vehicle (treatment group 2), COT inhibitor (Compound A referred to as TPL2i, 100 mg/Kg QD (low dose), 100 mg/Kg BID (high dose)), ACC inhibitor (Compound B referred to as ACCi, 10 mg/kg QD), and coadministration of Compound B with Compound A (treatment groups 5 and 6). Data in **FIG. 2** is based on all animals in the treatment group. In **FIG. 2****,** a higher value for PSR % area is reflective of a higher level of fibrosis.

As seen in **FIG. 2****,** after 42 days of QD and BID dosing Compound A in this model, fibrosis as measured by PSR was decreased by 41% (QD, treatment group 3, TPL2i low dose) and 55% (BID, treatment group 4, TPL2i high dose), respectively, relative to vehicle (treatment group 2) treated animals. Compound B reduced PSR by 33%, as is shown in **FIG. 2****.** Coadministration of Compound B with Compound A (treatment group 5 (TPL2i low dose + ACCi) and treatment group 6 (TPL2i High dose + ACCi)) did not further reduce fibrosis relative to single agent administration.

**FIG. 3** plots liver hydroxyproline (OH-P) content from liver tissue for control (treatment group 1), start and end for the vehicle (treatment group 2), COT inhibitor (Compound A referred to as TPL2i, low dose = 100 mg/Kg QD, high dose = 100 mg/Kg BID), ACC inhibitor (Compound B referred to as ACCi, 10 mg/kg QD), and coadministration of Compound B with Compound A (treatment group 5 (TPL2i low dose + ACCi) and treatment group 6 (TPL2i high dose + ACCi)). Liver hydroxyproline (OH-P) content was calculated from liver tissue samples. Briefly, cryo-powdered liver tissue was weighed and lysed in diH₂O using a bead-based TissueLyzer II homogenizer (Qiagen). Homogenates were clarified by centrifugation at 10000 x g at 4 °C for 10 min and protein concentrations were determined using a commercially-available bicinchoninic acid (BCA) colorimetric protein assay (Thermo Fisher Scientific, Cat. #: 23225). Protein was hydrolyzed and incubated at 110 °C overnight. Hydrolysates were brought to RT and centrifuged at 13000 x g for 5 min. A 7-point standard curve of OH-P was performed in duplicate for each assay. Hydrolysates and standards were added to separate wells of a 96-well plate and placed under vacuum at RT for ~5 h until completely desiccated. Chloramine T solution was added to each well and incubated at RT for 20 min on agitation. Then, Ehrlich's Reagent was added to each well and the plate was sealed and incubated at 65 °C for 20 min. The plate was brought to RT and absorbance was read at 560 nm using a Molecular Devices' SpectraMax 190 plate reader. Relative optical densities (O.D.s) were background-corrected against blank samples and standard curves for the conversion of O.D.s to concentration were generated using the 4-parameter curve-fit method. Unknown sample concentrations were determined using SoftMax Pro5 software (Molecular Devices) and corrected for sample dilutions as appropriate. Samples with O.D. values outside the linear range of the assay were repeated at an appropriate dilution.

As seen in **FIG. 3****,** BID treatment with Compound A was associated with a 38% reduction in liver hydroxyproline (treatment group 4) as compared to vehicle (treatment group 2) treated animals. Liver hydroxyproline levels were further reduced by 58% when Compound A was administered in combination with Compound B (treatment group 6).

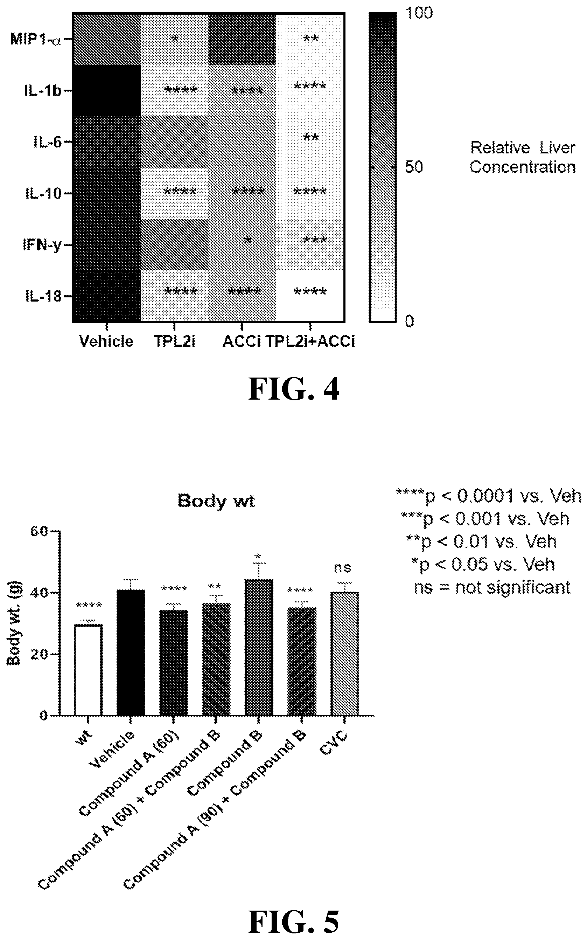

**FIG. 4** shows cytokine levels measured by ELISA from liver lysates of animals in treatment group 2 (vehicle), treatment group 4 (TPL2i), treatment group 6 (TPL2i + ACCi), and treatment group 7 (ACCi). Treatment with TPL2i resulted in statistically significant decreases in MIP1-α, IL-1b, IL-10 and IL-18. These effects were further reduced in combination with ACCi.

As shown in the above example, as well as **FIG. 1-4****,** Compound A reduced PSR and liver hydroxyproline. Co-administration with Compound B further reduced hydroxyproline with Compound A in a BID treatment.

### Example 2: Pharmacology Study with Compound A and Compound B Using the Gubra Amylin NASH (GAN) Model of Fatty Liver in Mice

The effect of COT inhibitor Compound A, ACC inhibitor Compound B, Cenicriviroc (CVC), and the combination of Compound A and Compound B in the Gubra amylin NASH mouse model (GAN) was determined.

**Table 2: Test Details**

| **Treatment group/ Diet** | **No. of animals** | **Test article** | **Dose mg/Kg** | **Dose Route** | **Regimen** | **Dose Vol (mL/Kg)** | **Dose (mg/mL)** |
|---|---|---|---|---|---|---|---|
| 1/ Chow | 10 | Vehicle | 0 | PO | BID, | 5 | 0 |
| 2/ GAN | 18 | Vehicle | 0 | PO | BID | 5 | 0 |
| 3/ GAN | 18 | Compound A | 60 | PO | BID | 5 | 12 |
| 4/ GAN | 18 | Compound A + Compound B | 60 + 10 | PO | BID + QD | 5 | 12 + 2 |
| 5/ GAN | 18 | Compound B | 10 | PO | QD | 5 | 2 |
| 6/ GAN | 18 | Cenicriviroc (CVC) | 100 | PO | BID | 5 | 20 |

| | | | | | | | |
|---|---|---|---|---|---|---|---|
| • Gubra Amylin NASH (GAN) diet: high in fat (40%), fructose (22%) and cholesterol (2%) (Research Diets D09100310)). • Vehicle: Copovidone and TPGS Vehicle (5% D-α-tocopherol polyethylene glycol succinate (TPGS), 10% copovidone (PVP-VA), 85% 0.01M HCl in deionized water (pH 2 water)). • Test articles were made in above vehicle. • Cenicriviroc (CVC) was used as a comparator compound. • Serum and Livers were collected at termination. | | | | | | | |

**FIG. 5** plots the body weight of treatment group 1 (wt), vehicle (treatment group 2), COT inhibitor (Compound A, 60 mg/kg BID), ACC inhibitor (Compound B, 10 mg/Kg QD), and combination of Compound A and Compound B (treatment group 4) in the Gubra amylin NASH (GAN) mouse model. Cenicriviroc (CVC) used as a control (100 mg/Kg QD).

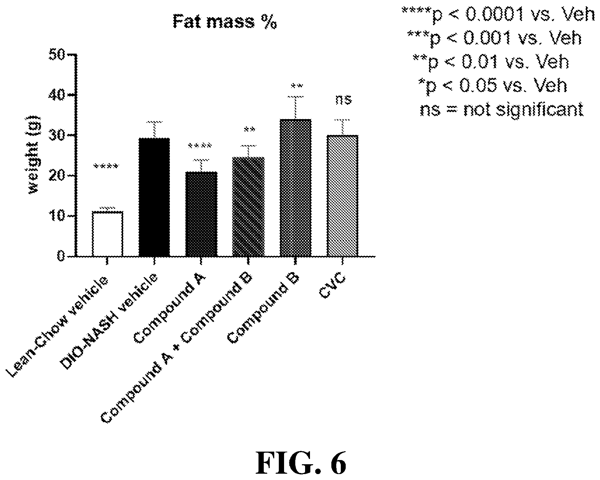

**FIG. 6** plots the percent fat mass of treatment group 1 (wt), vehicle (treatment group 2), COT inhibitor (Compound A, treatment group 3), ACC inhibitor (Compound B, treatment group 5), and combination of Compound A and Compound B (treatment group 4) in the Gubra amylin NASH (GAN) mouse model. Cenicriviroc (CVC) used as a control (treatment group 6). Fat mass was calculated by ECHO MRI.

After 84 days of dosing Compound A in this model, body weight and fat mass decreased by 16% and 31%, respectively **(****FIG. 5** and **FIG. 6****,** respectively).

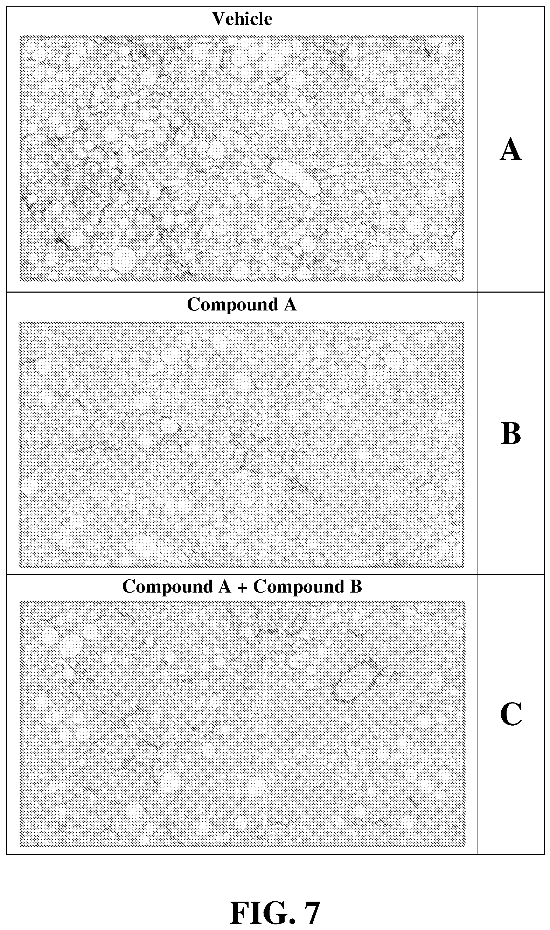

**FIG. 7** shows picrosirius red staining (PSR) from liver tissue from an animal in vehicle (treatment group 2, **FIG. 7** panel A), treatment group 3 (COT inhibitor, **FIG. 7** panel B), and treatment group 4 (ACC inhibitor, **FIG. 7** panel C).

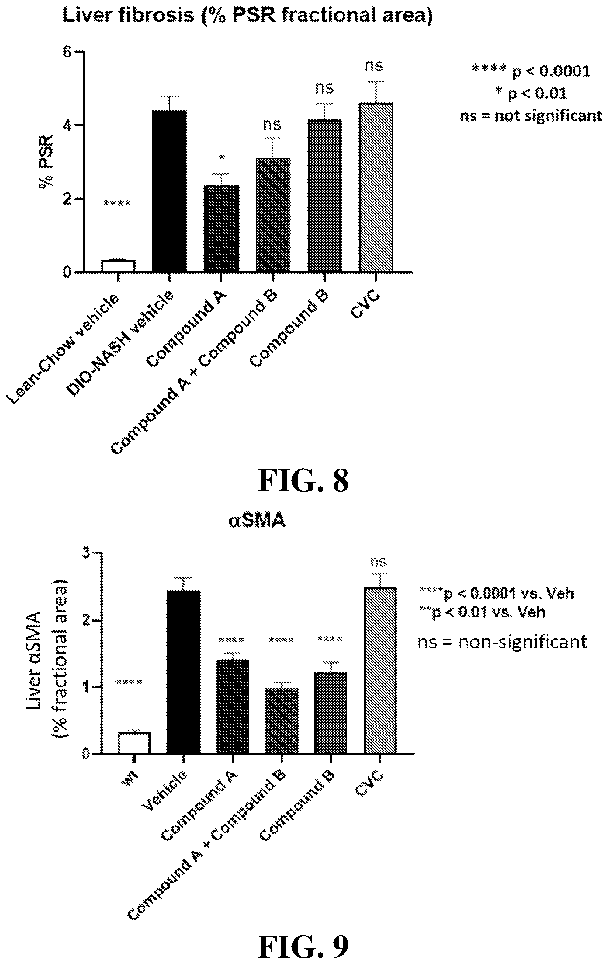

**FIG. 8** plots liver fibrosis levels based on percent PSR for treatment group 1 (wt), vehicle (treatment group 2), COT inhibitor (Compound A, treatment group 3), ACC inhibitor (Compound B, treatment group 5), and combination of Compound A and Compound B (treatment group 4) in the Gubra amylin NASH (GAN) mouse model. Cenicriviroc (CVC) used as a control (treatment group 6).

Fibrosis was measured by PSR and showed a significant decreased by 46% with Compound A as compared to vehicle (treatment group 2) **(****FIG. 7****,** panels A and B, respectively and **FIG. 8****).**

**FIG. 9** plots the anti-smooth muscle antibody (aSMA) levels from liver tissue of treatment group 1 (wt), vehicle (treatment group 2), COT inhibitor (Compound A, treatment group 3), ACC inhibitor (Compound B, treatment group 5), and combination of Compound A and Compound B (treatment group 4) in the Gubra amylin NASH (GAN) mouse model. Cenicriviroc (CVC) used as a control (treatment group 6). Alpha-smooth muscle actin (aSMA) levels were calculated from % area of alpha-SMA antibody staining on liver tissue (IHC).

Alpha-smooth muscle actin (aSMA) levels were reduced 41% with Compound A (treatment group 3) compared to vehicle (treatment group 2) **(****FIG. 9****).**

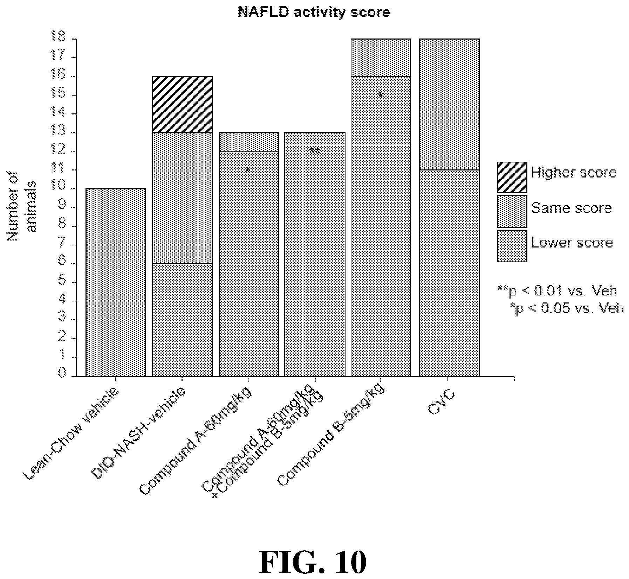

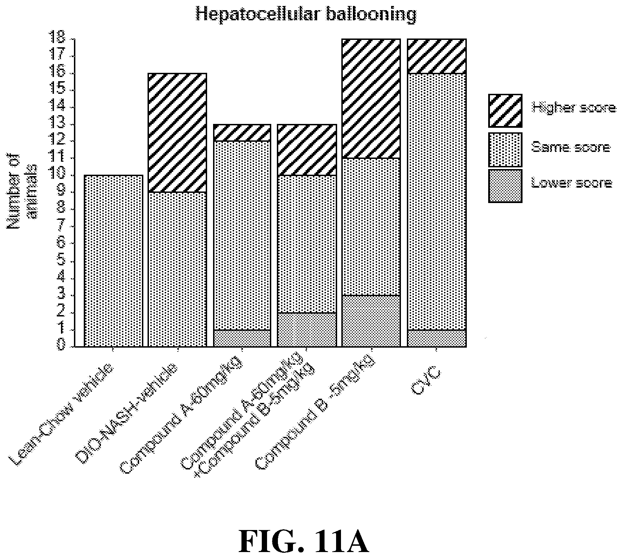

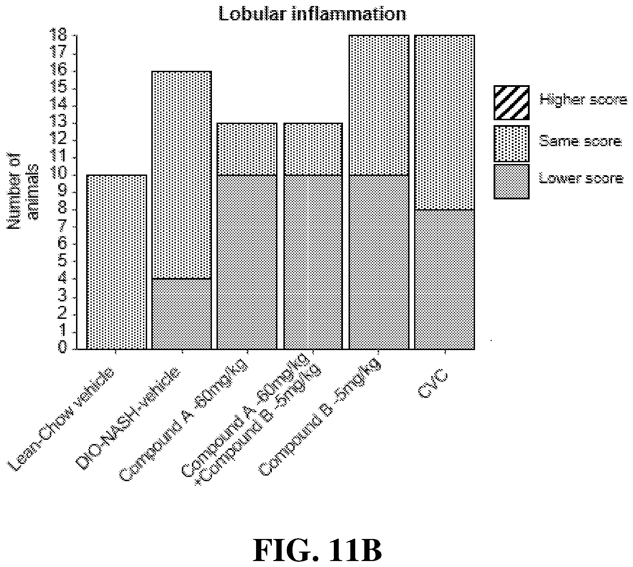

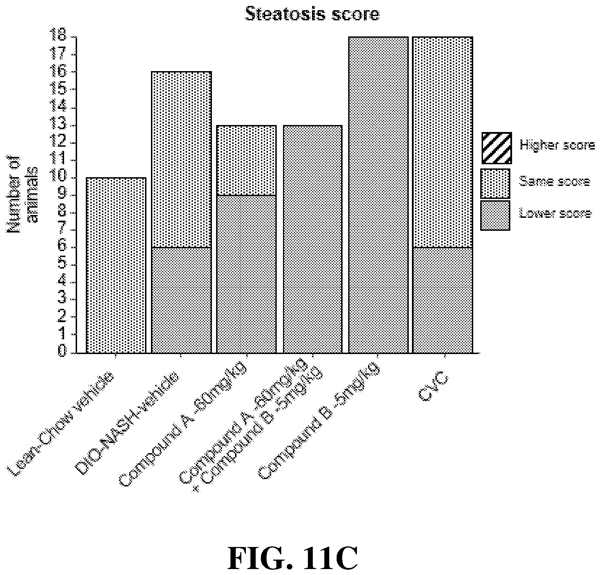

**FIG. 10** plots NAFLD activity score of Compound A (60 mg/kg BID), Compound B (10 mg/Kg QD), CVC (100 mg/Kg QD), and the combination Compound A and Compound B in from liver tissue the Gubra amylin NASH mouse model. CVC = Cenicriviroc, used as a control (100 mg/Kg QD). Individual components comprising the NAFLD score includes hepatocellular ballooning **(****FIG. 11A****),** lobular inflammation **(****FIG. 11B****),** and steatosis score **(****FIG. 11C****).**

NAFLD activity score was determined by scoring of steatosis, inflammation, and hepatocyte ballooning from hematoxylin and eosin stained liver sections. As shown in **FIG. 10****,** more animals had a lower NAFLD activity score at the end of the study when treated with Compound A as compared to vehicle.

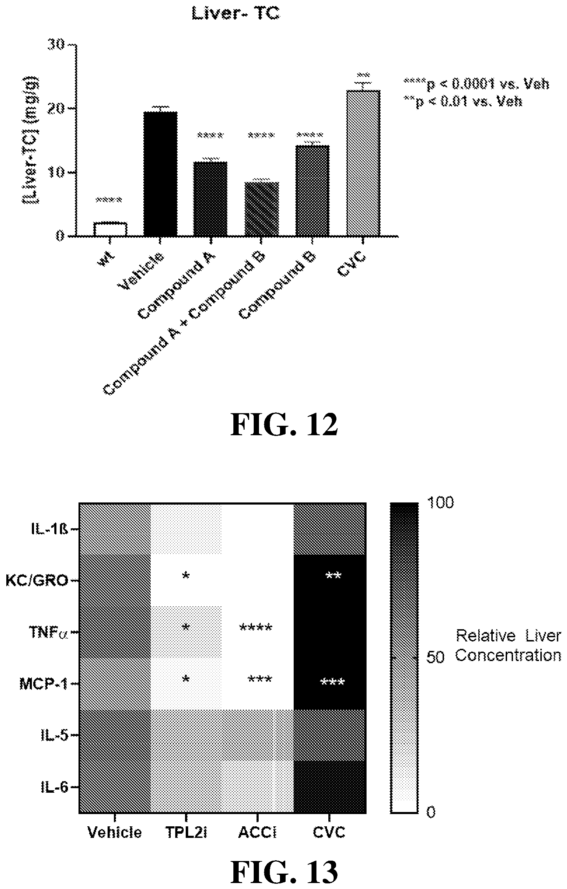

**FIG. 12** plots the liver total cholesterol levels for treatment group 1 (wt), vehicle (treatment group 2), COT inhibitor (Compound A, treatment group 3), ACC inhibitor (Compound B, treatment group 5), and combination of Compound A and Compound B (treatment group 4) in the Gubra amylin NASH (GAN) mouse model. Cenicriviroc (CVC) used as a control (treatment group 6). Total cholesterol levels were calculated from was measured using commercial kits (Roche Diagnostics) on the cobas c 501 autoanalyzer according to the manufacturer's instructions.

Liver total cholesterol **(****FIG. 12****)** was reduced by 40% by Compound A.

**FIG. 13** shows cytokine levels measured by ELISA from liver lysates of animals in treatment group 2 (vehicle), treatment group 3 (TPL2i), treatment group 5 (ACCi), and treatment group 6 (CVC). Treatment with TPL2i resulted in statistically significant decreases in IL-1β, KC/GRO, TNF-α, and MCP-1. Changes in cytokine levels were not observed with CVC.

The data obtained per the above example demonstrates that Compound A reduced PSR, aSMA levels and NAFLD activity score with BID treatment relative to vehicle in the Gubra amylin NASH (GAN) mouse model. The data obtained per the above example also demonstrates that Compound A reduced PSR with BID treatment relative to Compound B alone in the Gubra amylin NASH (GAN) mouse model. Compound A also decreased body weight, fat mass, and liver cholesterol relative to vehicle.

## Claims

1. A compound having the formula 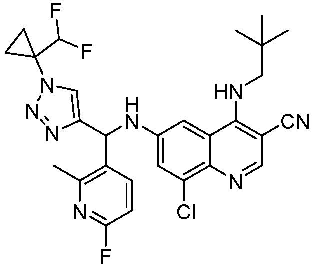 or a pharmaceutically acceptable salt thereof, or a pharmaceutical composition comprising the compound or a pharmaceutically acceptable salt thereof and one or more pharmaceutically acceptable vehicles selected from carriers, adjuvants and excipients, for use in a method for treating non-alcoholic steatohepatitis (NASH) in a subject.

2. The compound or pharmaceutical composition for use of claim 1, wherein the subject is a human.

3. The compound or pharmaceutical composition for use of claim 1 or 2, wherein the method further comprises administering one or more additional therapeutic agents.

4. The compound or pharmaceutical composition for use of any one of claims 1 to 3, wherein the method further comprises administering two or more additional therapeutic agents.

5. The compound or pharmaceutical composition for use of claim 1 or 2, wherein the method further comprises administering one or more additional therapeutic agents selected from agents useful for the treatment and/or prophylaxis of an inflammatory, metabolic, and/or fibrotic condition or disease.

6. The compound or pharmaceutical composition for use of claim 1 or 2, wherein the method further comprises administering one or more additional therapeutic agents selected from an apoptotic signal-regulating kinase (ASK-1) inhibitor, a farnesoid X receptor (FXR) agonist, a peroxisome proliferator-activated receptor alpha (PPARα) agonist, fish oil, an acetyl-coA carboxylase (ACC) inhibitor, a TGFβ antagonist, a LPAR antagonist, a SGLT2 inhibitor, a Tpl2 inhibitor, or a GLP-1 agonist, or a combination thereof.

7. The compound or pharmaceutical composition for use of claim 1 or 2, wherein the method further comprises administering an effective amount of an ACC inhibitor.

8. The compound or pharmaceutical composition for use of claim 7, wherein the ACC inhibitor is:

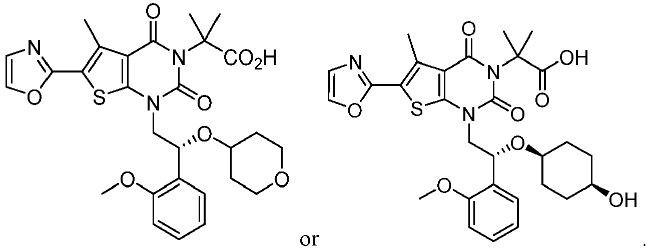

9. The compound or pharmaceutical composition for use of claim 7, wherein the ACC inhibitor is: 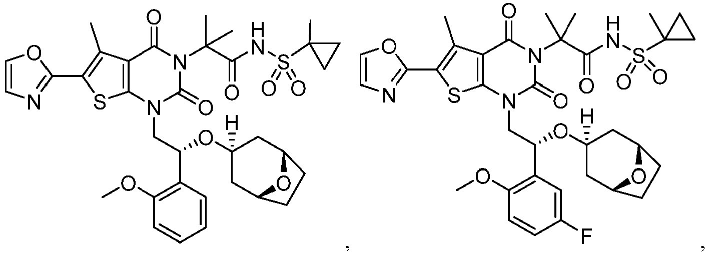 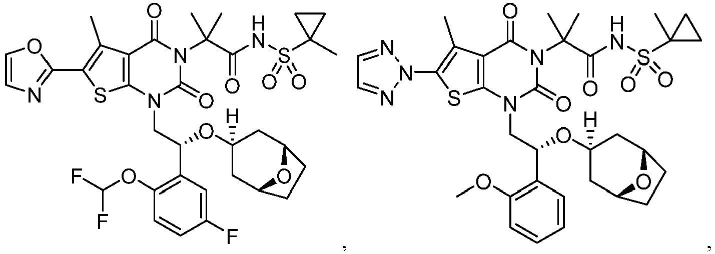 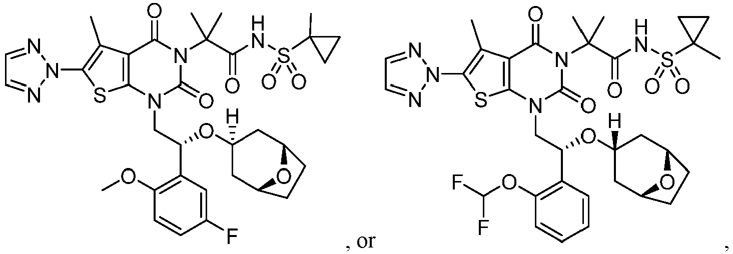 or a pharmaceutically acceptable salt thereof.

10. The compound or pharmaceutical composition for use of any one of claims 1 to 9, wherein the compound having the formula 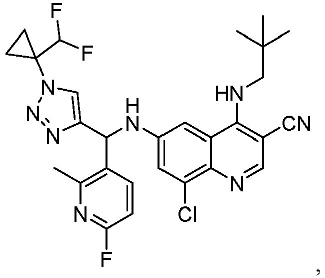 or a pharmaceutically acceptable salt thereof is administered intravenously, intraperitoneally, parenterally, intramuscularly, subcutaneously, orally, topically, as an inhalant, or by intra-arterial injection.

11. The compound or pharmaceutical composition for use of any one of claims 1 to 10, wherein the compound having the formula 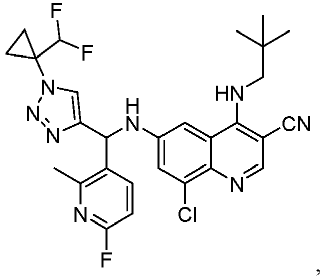 or a pharmaceutically acceptable salt thereof is administered at a dose in the range of 100 mg to 1500 mg per dose.

## Patentansprüche

1. Verbindung der Formel 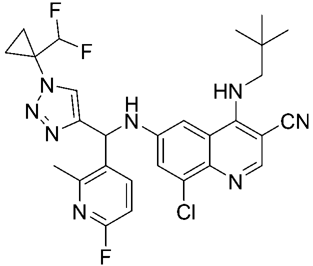 oder ein pharmazeutisch verträgliches Salz davon oder pharmazeutische Zusammensetzung, die die Verbindung oder ein pharmazeutisch verträgliches Salz davon und ein oder mehrere pharmazeutisch verträgliche Vehikel, ausgewählt aus Trägern, Adjuvantien und Hilfsstoffen, umfasst, zur Verwendung in einem Verfahren zum Behandeln von nicht-alkoholischer Steatohepatitis (NASH) bei einem Lebewesen.

2. Verbindung oder pharmazeutische Zusammensetzung zur Verwendung nach Anspruch 1, wobei das Lebewesen ein Mensch ist.

3. Verbindung oder pharmazeutische Zusammensetzung zur Verwendung nach Anspruch 1 oder 2, wobei das Verfahren ferner ein Verabreichen eines oder mehrerer zusätzlicher Therapeutika umfasst.

4. Verbindung oder pharmazeutische Zusammensetzung zur Verwendung nach einem jeglichen der Ansprüche 1 bis 3, wobei das Verfahren ferner ein Verabreichen von zwei oder mehr zusätzlichen Therapeutika umfasst.

5. Verbindung oder pharmazeutische Zusammensetzung zur Verwendung nach Anspruch 1 oder 2, wobei das Verfahren ferner ein Verabreichen eines oder mehrerer zusätzlicher Therapeutika, die aus Mitteln ausgewählt sind, die für die Behandlung und/oder Prophylaxe eines/einer entzündlichen, metabolischen und/oder fibrotischen Zustands oder Erkrankung nützlich sind, umfasst.

6. Verbindung oder pharmazeutische Zusammensetzung zur Verwendung nach Anspruch 1 oder 2, wobei das Verfahren ferner ein Verabreichen eines oder mehrerer zusätzlicher Therapeutika, ausgewählt aus einem Inhibitor der apoptotischen signalregulierenden Kinase (ASK-1), einem Farnesoid-X-Rezeptor (FXR)-Agonisten, einem Agonisten von Peroxisom-Proliferatoraktiviertem Rezeptor-alpha (PPARα), Fischöl, einem Inhibitor von Acetyl-CoA-Carboxylase (ACC), einem TGFβ-Antagonisten, einem LPAR-Antagonisten, einem SGLT2-Inhibitor, einem Tpl2-Inhibitor oder einem GLP-1-Agonisten oder einer Kombination davon, umfasst.

7. Verbindung oder pharmazeutische Zusammensetzung zur Verwendung nach Anspruch 1 oder 2, wobei das Verfahren ferner ein Verabreichen einer wirksamen Menge eines ACC-Inhibitors umfasst.

8. Verbindung oder pharmazeutische Zusammensetzung zur Verwendung nach Anspruch 7, wobei der ACC-Inhibitor: 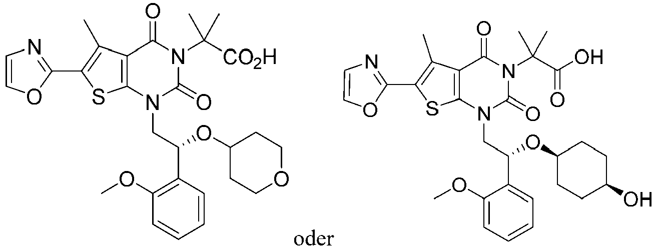 ist.

9. Verbindung oder pharmazeutische Zusammensetzung zur Verwendung nach Anspruch 7, wobei der ACC-Inhibitor: 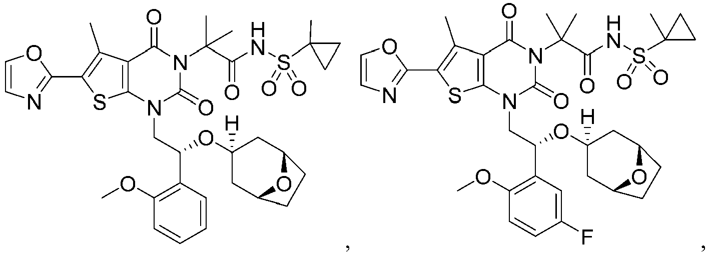 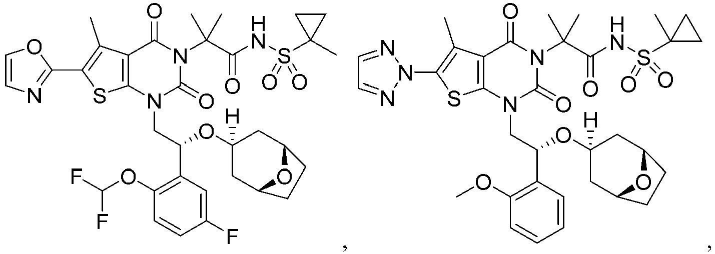 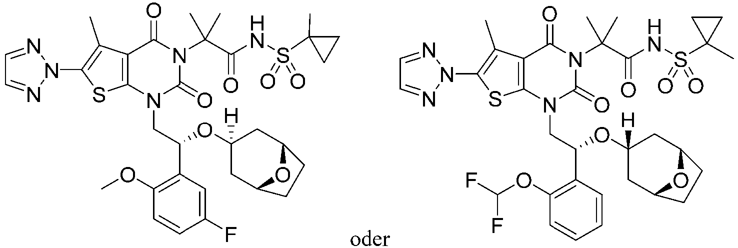 oder ein pharmazeutisch verträgliches Salz davon ist.

10. Verbindung oder pharmazeutische Zusammensetzung zur Verwendung nach einem jeglichen der Ansprüche 1 bis 9, wobei die Verbindung der Formel 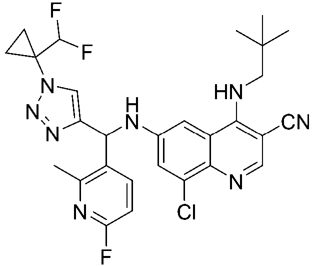 oder ein pharmazeutisch verträgliches Salz davon intravenös, intraperitoneal, parenteral, intramuskulär, subkutan, oral, topisch, als ein Inhalationsmittel oder durch intraarterielle Injektion verabreicht wird.

11. Verbindung oder pharmazeutische Zusammensetzung zur Verwendung nach einem jeglichen der Ansprüche 1 bis 10, wobei die Verbindung der Formel 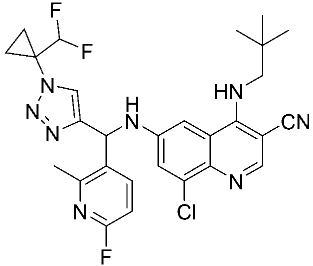 oder ein pharmazeutisch verträgliches Salz davon in einer Dosis in dem Bereich von 100 mg bis 1500 mg pro Dosis verabreicht wird.

## Revendications

1. Composé présentant la formule : 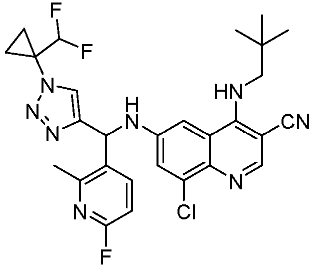 ou sel pharmaceutiquement acceptable de celui-ci, ou composition pharmaceutique comprenant le composé ou un sel pharmaceutiquement acceptable de celui-ci et un ou plusieurs véhicules pharmaceutiquement acceptables choisis parmi les vecteurs, les adjuvants et les excipients, destiné à être utilisé dans une méthode de traitement de la stéatohépatite non alcoolique (NASH) chez un sujet.

2. Composé ou composition pharmaceutique destiné à être utilisé selon la revendication 1, ledit sujet étant un humain.

3. Composé ou composition pharmaceutique destiné à être utilisé selon la revendication 1 ou 2, ladite méthode comprenant en outre l'administration d'un ou de plusieurs agents thérapeutiques supplémentaires.

4. Composé ou composition pharmaceutique destiné à être utilisé selon l'une quelconque des revendications 1 à 3, ladite méthode comprenant en outre l'administration de deux agents thérapeutiques supplémentaires ou plus.

5. Composé ou composition pharmaceutique destiné à être utilisé selon la revendication 1 ou 2, ladite méthode comprenant en outre l'administration d'un ou de plusieurs agents thérapeutiques supplémentaires choisis parmi des agents utiles pour le traitement et/ou la prophylaxie d'un état ou d'une maladie inflammatoire, métabolique et/ou fibrotique.

6. Composé ou composition pharmaceutique destiné à être utilisé selon la revendication 1 ou 2, ladite méthode comprenant en outre l'administration d'un ou de plusieurs agents thérapeutiques supplémentaires choisis parmi un inhibiteur de la kinase régulatrice du signal d'apoptose (ASK-1), un agoniste du récepteur farnesoïde X (FXR), un agoniste du récepteur alpha activé par proliférateur de peroxysome (PPARα), l'huile de poisson, un inhibiteur de l'acétyl-coA carboxylase (ACC), un antagoniste du TGFβ, un antagoniste du LPAR, un inhibiteur du SGLT2, un inhibiteur du Tpl2 ou un agoniste du GLP-1, ou une combinaison de ceux-ci.

7. Composé ou composition pharmaceutique destiné à être utilisé selon la revendication 1 ou 2, ladite méthode comprenant en outre l'administration d'une quantité efficace d'un inhibiteur de l'ACC.

8. Composé ou composition pharmaceutique destiné à être utilisé selon la revendication 7, ledit inhibiteur de l'ACC étant :

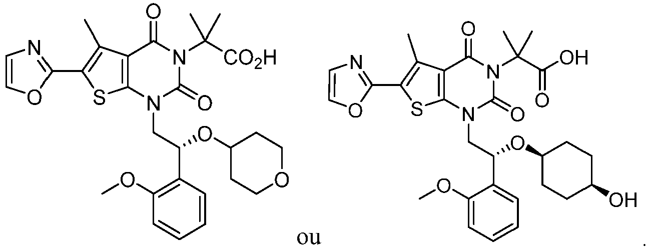

9. Composé ou composition pharmaceutique destiné à être utilisé selon la revendication 7, ledit inhibiteur de l'ACC étant : 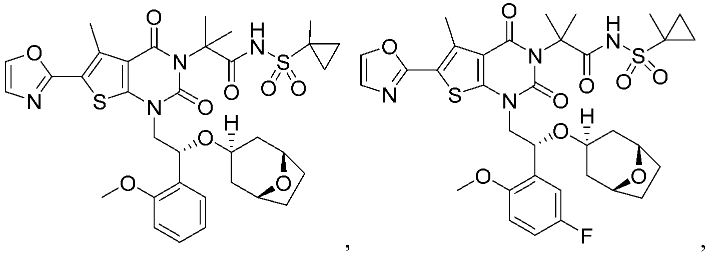 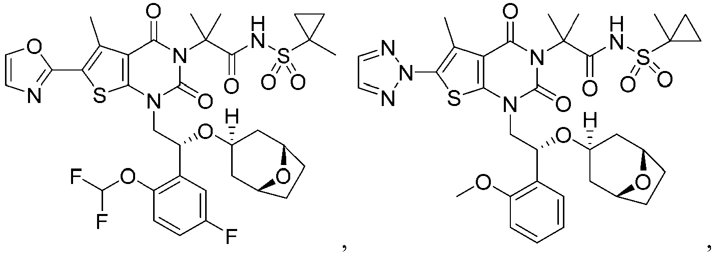 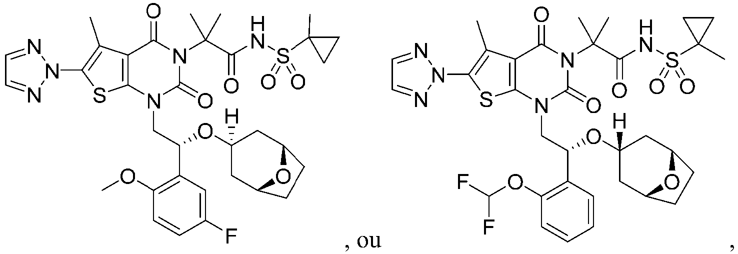 ou un sel pharmaceutiquement acceptable de ceux-ci.

10. Composé ou composition pharmaceutique destiné à être utilisé selon l'une quelconque des revendications 1 à 9, ledit composé présentant la formule 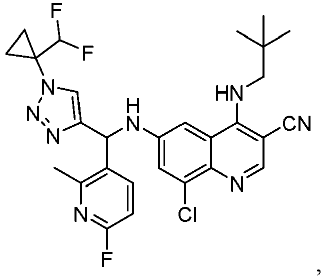 ou un sel pharmaceutiquement acceptable de celui-ci étant administré par voie intraveineuse, intrapéritonéale, parentérale, intramusculaire, sous-cutanée, orale, topique, par inhalation, ou par injection intra-artérielle.

11. Composé ou composition pharmaceutique destiné à être utilisé selon l'une quelconque des revendications 1 à 10, ledit composé présentant la formule 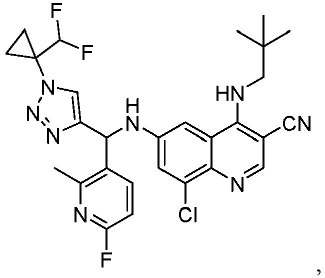 ou un sel pharmaceutiquement acceptable de celui-ci étant administré à une dose comprise dans la plage de 100 mg à 1500 mg par dose.
